# EUROPEAN PATENT SPECIFICATION

(11) **EP 3 643 712 B1**
(45) Date of publication and mention of the grant of the patent: **22.03.2023**
(21) Application number: 19203787.7
(22) Date of filing: 17.10.2019
(51) Int. Cl.: C07D 409/14, H01L 51/00

(54) **COMPOUND AND FILM AND PHOTOELECTRIC DIODE AND ORGANIC SENSOR AND ELECTRONIC DEVICE**
VERBINDUNG UND FILM SOWIE FOTOELEKTRISCHE DIODE UND ORGANISCHER SENSOR UND ELEKTRONISCHE VORRICHTUNG
COMPOSÉ, FILM, DIODE PHOTOÉLECTRIQUE, CAPTEUR ORGANIQUE ET DISPOSITIF ÉLECTRONIQUE

(30) Priority: 24.10.2018 KR 20180127734
(43) Date of publication of application: 29.04.2020
(73) Proprietor: Samsung Electronics Co., Ltd., Gyeonggi-do 16677 (KR)
(72) Inventor: KWON, Ohkyu, Seoul (KR); CHOI, Hyesung, Seoul (KR); PARK, Bum Woo, Gyeonggi-do (KR); KIM, Hwang Suk, Gyeonggi-do (KR); RO, Takkyun, Gyeonggi-do (KR); LEE, Kwang Hee, Gyeonggi-do (KR); LEEM, Dong-Seok, Gyeonggi-do (KR)
(74) Representative: Elkington and Fife LLP

(56) References cited:
- EP-A1- 0 574 118
- EP-A1- 2 671 880
- JOS? P. CER?N-CARRASCO ET AL: "Excited-state nature in benzodifuranone dyes: Insights from ab initio simulations", DYES AND PIGMENTS, vol. 92, no. 3, 1 March 2012 (2012-03-01), pages 1144-1152, XP055030417, ISSN: 0143-7208, DOI: 10.1016/j.dyepig.2011.07.016

## Description

### FIELD OF THE INVENTION

Compounds, film, photoelectric diodes, organic sensors, and electronic devices are disclosed.

### BACKGROUND OF THE INVENTION

Imaging devices are used in digital cameras, camcorders, etc., to capture an image and to store the captured image as an electrical signal, and imaging devices include an image sensor that may separate incident light into separate components defined by separate wavelength spectrums and convert each separate component to an electrical signal.

Recently, photoelectric diodes configured to detect light in the near infra-red and infrared wavelength spectrum have been researched to improve sensitivity of a sensor in a low-illumination environment or for use as in biometric devices.

J. P. Cerón-Carrasco et al. in "Excited-state nature in benzodifuranone dyes: Insights from ab initio simulations", Dyes and Pigments, Volume 92, Issue 3, 2012, simulated the optical spectra of benzodifuranone dyes using ab initio theoretical tools, simultaneously accounting for electron correlation and environmental effects.

EP 2 671 880 discloses a low-molecular-weight fused polycyclic heteroaromatic compound having a compact planar structure in which seven or more rings are fused together. An organic thin film and electronic device including the fused polycyclic heteroaromatic compound are also disclosed.

EP 0 574 118 discloses a benzodifuranone dye containing at least one thiazolyl group.

### SUMMARY OF THE INVENTION

Some example embodiments provide a compound that is chemically tuned to exhibit good light absorption characteristics for a chosen wavelength spectrum, like the near infra-red spectrum.

Some example embodiments provide a film including the compound.

Some example embodiments provide a photoelectric diode including the compound.

Some example embodiments provide an organic sensor including the compound or the photoelectric diode.

Some example embodiments provide an electronic device including the photoelectric diode or the organic sensor.

According to an aspect of the invention, a compound represented by Chemical Formula 1 is provided in accordance with claim 1.

Ar may be a substituted or unsubstituted benzene, a substituted or unsubstituted naphthalene, a substituted or unsubstituted anthracene, a substituted or unsubstituted phenanthrene, a substituted or unsubstituted tetracene, a substituted or unsubstituted pyrene, a substituted or unsubstituted thiophene, a substituted or unsubstituted furan, a substituted or unsubstituted selenophene, a substituted or unsubstituted tellurophene, or a fused ring of two or more of these.

R¹ may be represented by one of Chemical Formulae A-1 to A-3. 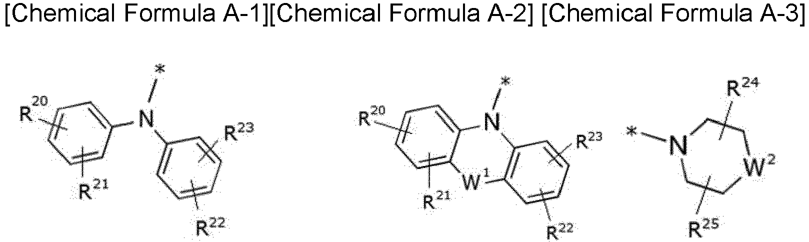 W¹ is a single bond, O, S, Se, NR^{e}, CR^{f}R^{g}, or SiR^{h}Rⁱ,
W² is O, S, Se, NR^{e}, CR^{f}R^{g} or SiR^{h}Rⁱ,
R²⁰ to R²⁵ and R^{e} to Rⁱ are independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof,
R²⁰ and R²¹ may each be independently present or linked with each other to form a ring,
R²² and R²³ may each be independently present or linked with each other to form a ring,
R²⁴ and R²⁵ may each be independently present or linked with each other to form a ring,
R^{f} and R^{g} may each be independently present or linked with each other to form a ring,
R^{h} and Rⁱ may each be independently present or linked with each other to form a ring,
in Chemical Formula A-1, Chemical Formula A-2, or Chemical Formula A-3, at least one hydrogen in the ring structure may be replaced by a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted silyl group, a halogen or a combination thereof, and
* is a linking point.
R⁴ may be represented by one of Chemical Formulae B-1 to B-3.

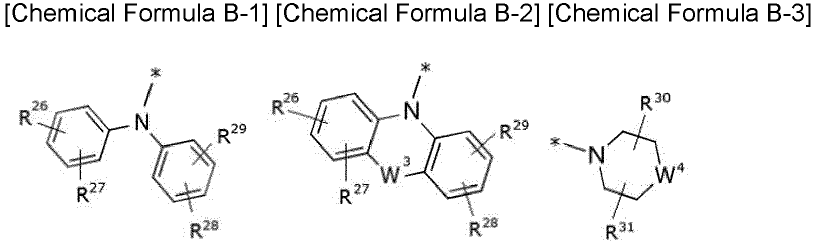

In Chemical Formulae B-1 to B-3,
W³ is a single bond, O, S, Se, NR^{j}, CR^{k}R^{l} or SiR^{m}Rⁿ,
W⁴ is O, S, Se, NR^{j}, CR^{k}R^{l} or SiR^{m}Rⁿ,
R²⁶ to R³¹ and R^{j} to Rⁿ are independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof,
R²⁶ and R²⁷ may each be independently present or linked with each other to form a ring,
R²⁸ and R²⁹ may each be independently present or linked with each other to form a ring,
R³⁰ and R³¹ may each be independently present or linked with each other to form a ring,
R^{k} and R^{l} may each be independently present or linked with each other to form a ring,
R^{m} and Rⁿ may each be independently present or linked with each other to form a ring,
in Chemical Formula B-1, Chemical Formula B-2, or Chemical Formula B-3, at least one hydrogen in the ring structure may be replaced by a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted silyl group, a halogen, or a combination thereof, and
* is a linking point.

The compound may be represented by one of Chemical Formulae 1-J to 1-Z2.

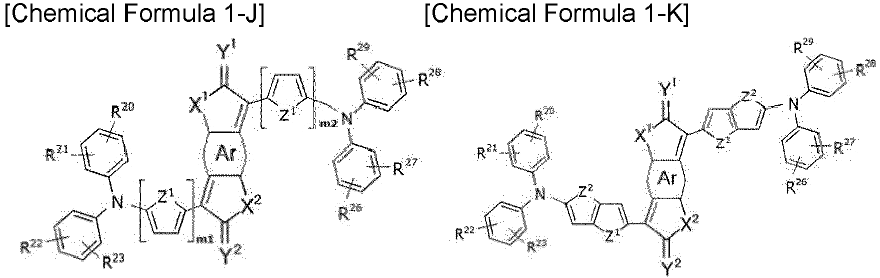

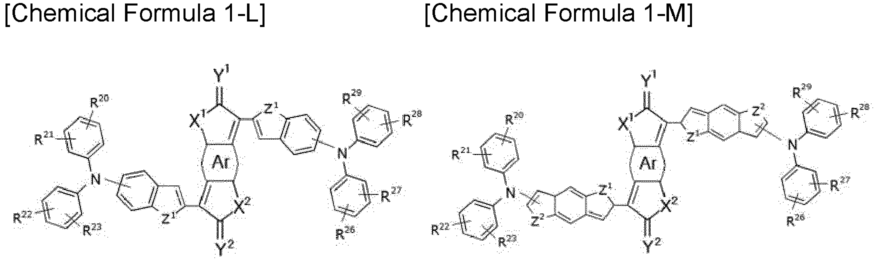

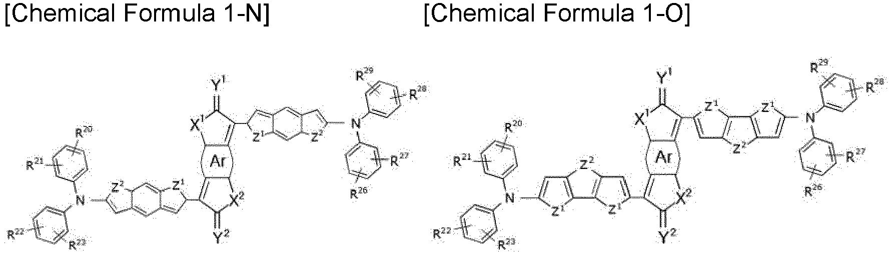

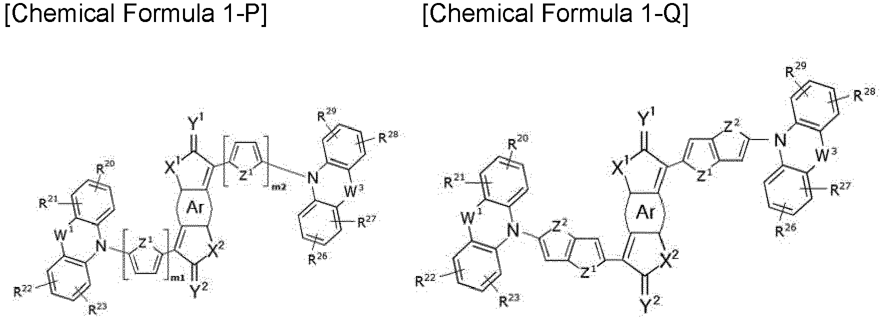

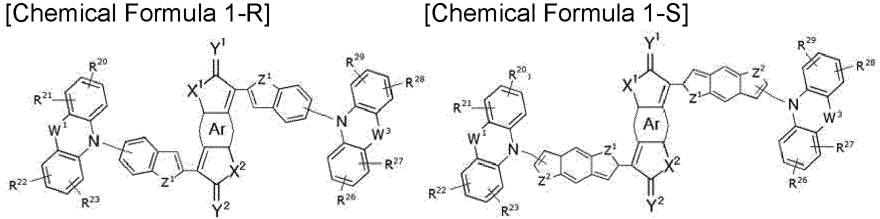

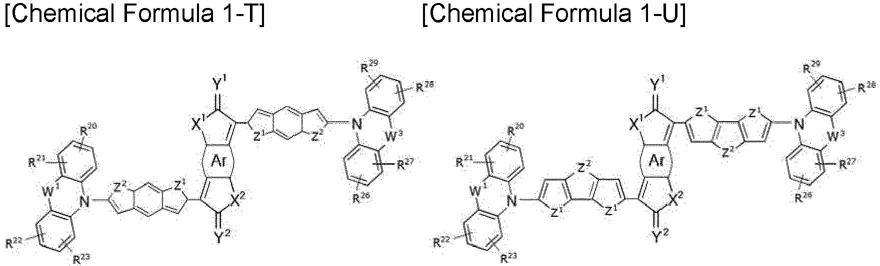

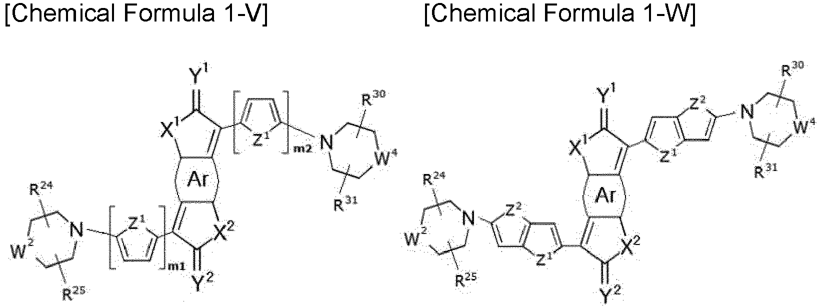

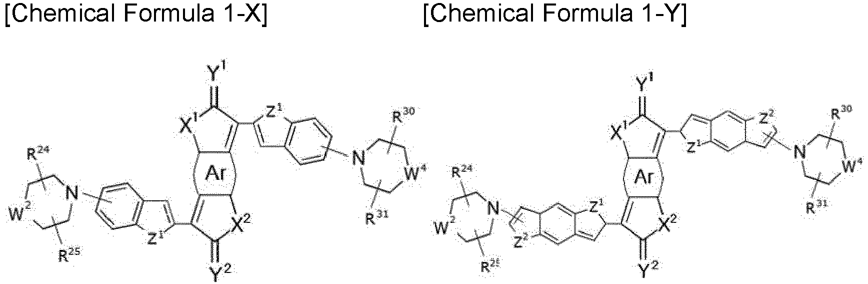

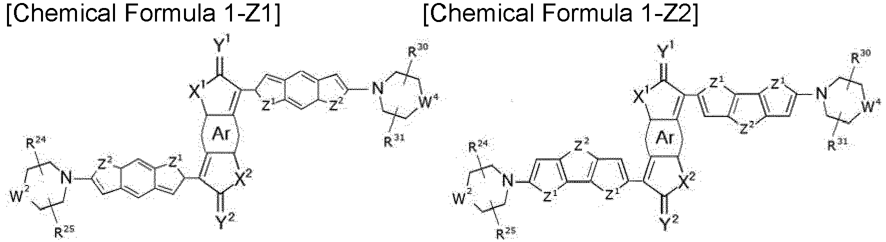

In Chemical Formulae 1-J to 1-Z2,
X¹ and X² are independently O, S, Se, or Te,
Y¹ and Y² are independently O, S, Se, Te, or C(CN)₂,
Ar is a substituted or unsubstituted C6 to C30 aromatic ring, a substituted or unsubstituted C3 to C30 heteroaromatic ring, or a combination thereof,
Z¹ and Z² are independently O, S, Se, Te, NR^{a}, or SiR^{b}R^{c},
W¹ is a single bond, O, S, Se, NR^{e}, CR^{f}R^{g}, or SiR^{h}Rⁱ,
W² is O, S, Se, NR^{e}, CR^{f}R^{g}, or SiR^{h}Rⁱ,
W³ is a single bond, O, S, Se, NR^{j}, CR^{k}R^{l}, or SiR^{m}Rⁿ,
W⁴ is O, S, Se, NR^{j}, CR^{k}R^{l}, or SiR^{m}Rⁿ,
R²⁰ to R³¹ and R^{a} to Rⁿ are independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof,
R^{b} and R^{c} may each be independently present or linked with each other to form a ring,
R^{f} and R^{g} may each be independently present or linked with each other to form a ring,
R^{h} and Rⁱ may each be independently present or linked with each other to form a ring,
R^{k} and R^{l} may each be independently present or linked with each other to form a ring,
R^{m} and Rⁿ may each be independently present or linked with each other to form a ring, and
m1 and m2 are independently an integer from 1 to 5.

A peak absorption wavelength of the compound may be within a wavelength spectrum of 700 nm to 3000 nm.

According to another embodiment, a film including the compound is provided.

According to another aspect of the invention, a photoelectric diode is provided in accordance with claim 8.

According to some example embodiments, an organic sensor including the photoelectric diode is provided.

According to some example embodiments, an electronic device including the photoelectric diode or the organic sensor is provided.

The compound may have good light absorption characteristics in a near infra-red wavelength spectrum and thus may be effectively used in a photoelectric diodes and/or an organic sensor.

### BRIEF DESCRIPTION OF THE DRAWINGS

FIG. 1 is a schematic view showing an example of a pixel array of a sensor according to some example embodiments.
FIG. 2 is a cross-sectional view showing a photoelectric diode according to some example embodiments,
FIG. 3 is a cross-sectional view showing an organic sensor according to some example embodiments,
FIG. 4 is a cross-sectional view showing an organic sensor according to some example embodiments,
FIG. 5 is a cross-sectional view showing an organic sensor according to some example embodiments,
FIG. 6 is a schematic cross-sectional view of an organic sensor according to some example embodiments,
FIG. 7 is a schematic cross-sectional view of an organic sensor according to some example embodiments,
FIG. 8 is a cross-sectional view showing an organic sensor according to some example embodiments,
FIG. 9 is a perspective view of an organic sensor according to some example embodiments,
FIG. 10 is a schematic cross-sectional view showing the organic sensor of FIG. 9 according to some example embodiments,
FIG. 11 is a schematic cross-sectional view of an organic sensor according to some example embodiments,
FIG. 12 is a perspective view of an organic sensor according to some example embodiments,
FIG. 13 is a schematic cross-sectional view showing the organic sensor of FIG. 12 according to some example embodiments,
FIG. 14 is a schematic diagram of an electronic device according to some example embodiments, and
FIG. 15 is a graph showing photoelectric conversion efficiency of the photoelectric diode according to Example.

### DETAILED DESCRIPTION OF THE EMBODIMENTS

Example embodiments will hereinafter be described in detail, and may be easily performed by those who have common knowledge in the related art. However, actually applied structures may be embodied in many different forms and is not construed as limited to the exemplary embodiments set forth herein.

In the drawings, the thickness of layers, films, panels, spectrums, etc., are not drawn to scale and are exaggerated for clarity.

When the terms "about" or "substantially" are used in this specification in connection with a numerical value, it is intended that the associated numerical value include a tolerance of ±10% around the stated numerical value. When ranges are specified, the range includes all values therebetween such as increments of 0.1%.

It will be understood that when an element such as a layer, film, spectrum, or substrate is referred to as being "on" another element, it may be directly on the other element or intervening elements may also be present. In contrast, when an element is referred to as being "directly on" another element, there are no intervening elements present.

As used herein, when a definition is not otherwise provided, "substituted" refers to replacement of hydrogen of a compound or a group by a substituent selected from a halogen atom, a hydroxy group, a nitro group, a cyano group, an amino group, an azido group, an amidino group, a hydrazino group, a hydrazono group, a carbonyl group, a carbamyl group, a thiol group, an ester group, a carboxyl group or a salt thereof, a sulfonic acid group or a salt thereof, a phosphoric acid group or a salt thereof, a silyl group, a C1 to C20 alkyl group, a C2 to C20 alkenyl group, a C2 to C20 alkynyl group, a C6 to C30 aryl group, a C7 to C30 arylalkyl group, a C1 to C30 alkoxy group, a C1 to C20 heteroalkyl group, a C3 to C20 heteroaryl group, a C3 to C20 heteroarylalkyl group, a C3 to C30 cycloalkyl group, a C3 to C15 cycloalkenyl group, a C6 to C15 cycloalkynyl group, a C3 to C30 heterocycloalkyl group, and a combination thereof.

As used herein, when a definition is not otherwise provided, "hetero" refers to one including 1 to 4 heteroatoms selected from N, O, S, Se, Te, Si, and P.

As used herein, when a definition is not otherwise provided, "aromatic ring" refers to a functional group in which all atoms in the cyclic functional group have a p-orbital, wherein these p-orbitals are conjugated. In some example embodiments, the aromatic ring may be a C6 to C30 aryl group or a C2 to C30 heteroaryl group.

As used herein, when a definition is not otherwise provided, "aryl group" refers to a group including at least one aromatic hydrocarbon moiety. All elements of the aromatic hydrocarbon moiety have p-orbitals which form conjugation, for example a phenyl group, a naphthyl group, and the like, two or more aromatic hydrocarbon moieties may be linked by a sigma bond and may be, for example a biphenyl group, a terphenyl group, a quarterphenyl group, and the like, and two or more aromatic hydrocarbon moieties may be fused directly or indirectly to provide a non-aromatic fused ring, for example a fluorenyl group. The aryl group may include a monocyclic, polycyclic or fused polycyclic (i.e., rings sharing adjacent pairs of carbon atoms) functional group.

As used herein, when a definition is not otherwise provided, "heteroaryl group" includes at least one heteroatom selected from N, O, S, Se, Te, P, and Si instead of carbon (C) in the ring. When the heteroaryl group is a fused ring, at least one of the ring constituting the heteroaryl group may have a heteroatom, for example, each ring may have a heteroatom.

As used herein, when a definition is not otherwise provided, "ring" refers to an aromatic ring, non-aromatic ring, hetero aromatic ring, hetero non-aromatic ring, fused ring, and/or a combination thereof. The aromatic ring may be a C6 to C30 aryl group or a C2 to C30 heteroaryl group and the non-aromatic ring may be a C3 to C30 cycloalkyl group, a C3 to C30 cycloalkenyl group, a C3 to C30 cycloalkynyl group, a C3 to C30 heterocycloalkyl group, a C3 to C30 heterocycloalkenyl group, or a C3 to C30 heterocycloalkynyl group.

Hereinafter, a compound according to an embodiment is described.

The compound according to the invention is represented by Chemical Formula 1.

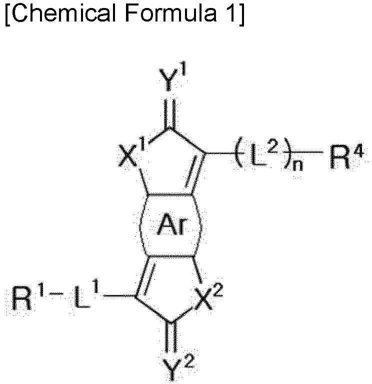

In Chemical Formula 1,
X¹ and X² are each independently O, S, Se or Te,
Y¹ and Y² are each independently O, S, Se, Te or C(CN)₂,
Ar is a substituted or unsubstituted C6 to C30 aromatic ring, a substituted or unsubstituted C3 to C30 heteroaromatic ring, or a combination thereof,
L¹ and L² are each independently a substituted or unsubstituted C3 to C20 heteroarylene group,
R¹ is -NR²R³, wherein R² and R³ are each independently hydrogen, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted silyl group, a halogen, or a combination thereof, wherein R² and R³ are each independently present or linked with each other to form a ring,
R⁴ is represented by -NR⁵R⁶,
R⁵ and R⁶ are each independently hydrogen, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted silyl group, a halogen, or a combination thereof, wherein R⁵ and R⁶ are each independently present or linked with each other to form a ring, and
n is 0 or 1.

The compound may have good electrical characteristics as well as light absorption characteristics configured to effectively absorb light in the near infra-red wavelength spectrum by combining substituted or unsubstituted amine groups having electron donating characteristics with the core of a conjugation structure having an electron accepting characteristic via a heteroarylene group as a linker.

A peak absorption wavelength (λₘₐₓ) of the compound may be for example greater than or equal to 700 nm. The peak absorption wavelength of the compound may be for example within a wavelength spectrum of 700 nm to 3000 nm.

In some example embodiments, X¹ and X² may be the same.

In some example embodiments, X¹ and X² may be different from each other.

In some example embodiments, X¹ and X² may each independently be O or S.

In some example embodiments, Y¹ and Y² may be the same.

In some example embodiments, Y¹ and Y² may be different from each other.

In some example embodiments, Y¹ and Y² may each independently be O, S, or C(CN)₂.

In some example embodiments, X¹ and Y¹ may be the same and X² and Y² may be the same.

In some example embodiments, X¹ and Y¹ may be different from each other and X² and Y² may be different from each other.

In some example embodiments, Ar may be a substituted or unsubstituted benzene, a substituted or unsubstituted naphthalene, a substituted or unsubstituted anthracene, a substituted or unsubstituted phenanthrene, a substituted or unsubstituted tetracene, a substituted or unsubstituted pyrene, a substituted or unsubstituted thiophene, a substituted or unsubstituted furan, a substituted or unsubstituted selenophene, a substituted or unsubstituted tellurophene, or a fused ring of two or more of these.

In some example embodiments, L¹ and L² are each independently at least one of substituted or unsubstituted groups listed in Group 1.

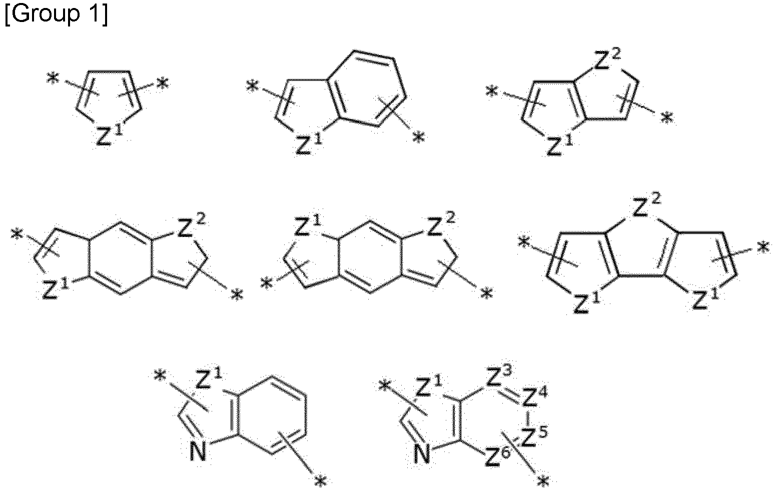

In Group 1,
Z¹ and Z² are each independently O, S, Se, Te, NR^{a}, or SiR^{b}R^{c},
Z³ to Z⁶ are each independently N or CR^{d},
at least one of Z³ to Z⁶ is N,
R^{a} to R^{d} are independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof, wherein R^{b} and R^{c} are each independently present or linked with each other to form a ring.

Each group in Group 1 may be unsubstituted or substituted with one or more substituents. Herein, the substituent may be for example a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof, but is not limited thereto.

In some example embodiments, L¹ and L² may each independently include at least one of substituted or unsubstituted groups listed in Group 2, but are not limited thereto.

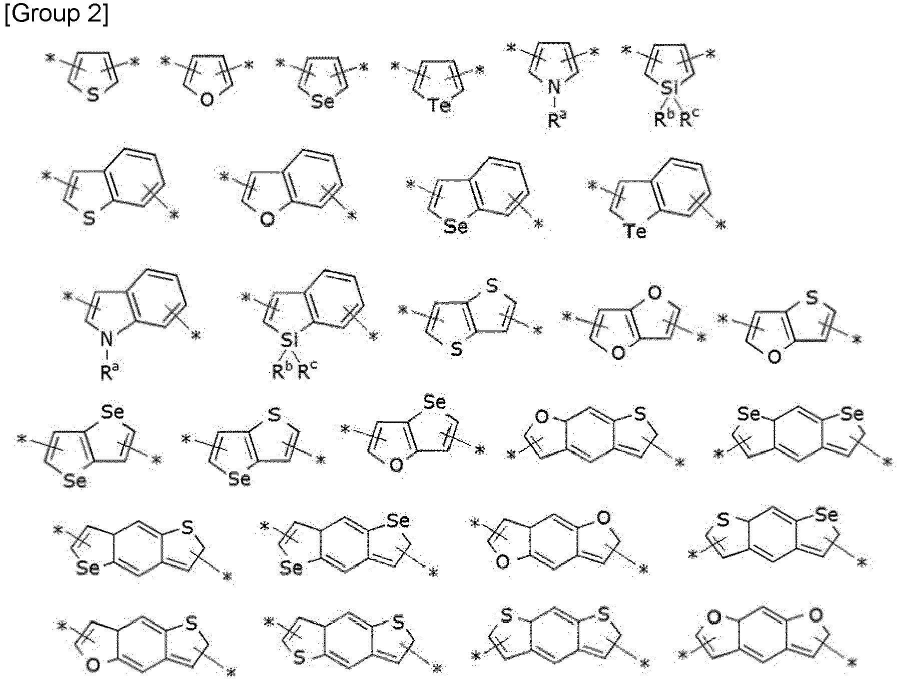

In Group 2,
R^{a} to R^{c} are each independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof, wherein R^{b} and R^{c} are are each independently present or linked with each other to form a ring.

In some example embodiments, the compound may be represented by one of Chemical Formulae 1-A to 1-F according to L¹ and L², but is not limited thereto.

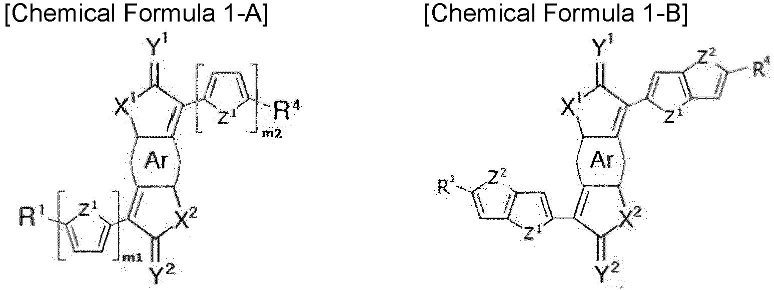

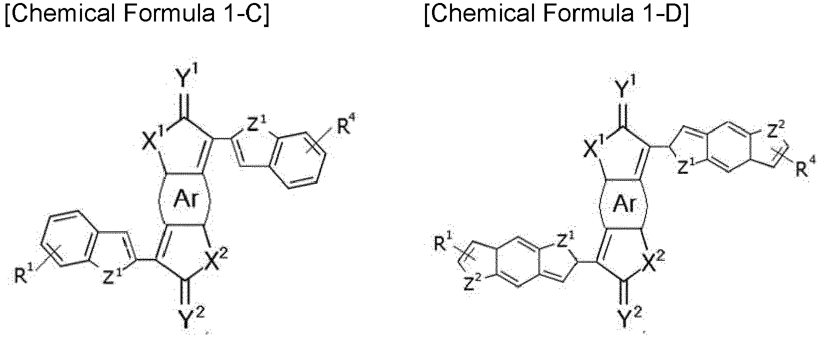

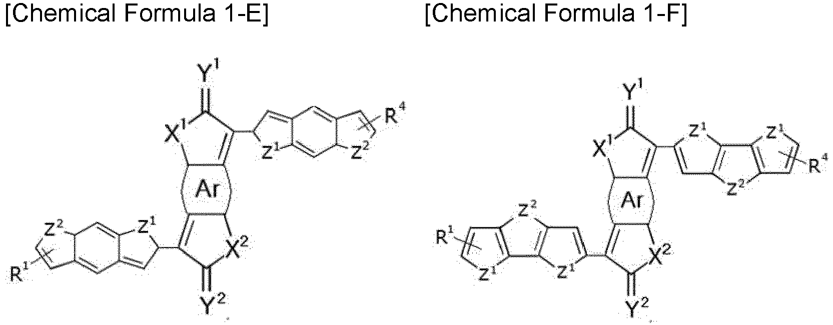

In Chemical Formulae 1-A to 1-F,
X¹, X², Y¹, Y², Ar, Z¹, Z², R¹, and R⁴ are the same as described above, and
m1 and m2 may each independently be an integer from 1 to 5.

In some example embodiments, R¹ may be represented by one of Chemical Formulae A-1 to A-3.

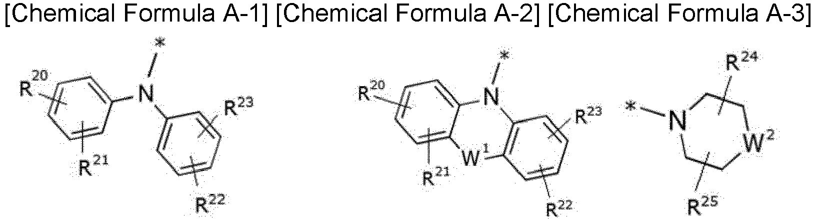

In Chemical Formulae A-1 to A-3,
W¹ is a single bond, O, S, Se, NR^{e}, CR^{f}R^{g}, or SiR^{h}Rⁱ,
W² is O, S, Se, NR^{e}, CR^{f}R^{g}, or SiR^{h}Rⁱ,
R²⁰ to R²⁵ and R^{e} to Rⁱ are each independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof,
R²⁰ and R²¹ may each be independently present or linked with each other to form a ring,
R²² and R²³ may each be independently present or linked with each other to form a ring,
R²⁴ and R²⁵ may each be independently present or linked with each other to form a ring,
R^{f} and R^{g} may each be independently present or linked with each other to form a ring,
R^{h} and Rⁱ may each be independently present or linked with each other to form a ring, and
* is a linking point.

In Chemical Formula A-1, Chemical Formula A-2, or Chemical Formula A-3, at least one hydrogen in the ring structure may be replaced by a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted silyl group, a halogen, or a combination thereof.

In some example embodiments, the compound may include two amine groups, that is, R¹ is a substituted or unsubstituted amine group.

In some example embodiments, R⁴ may be represented by one of Chemical Formulae B-1 to B-3.

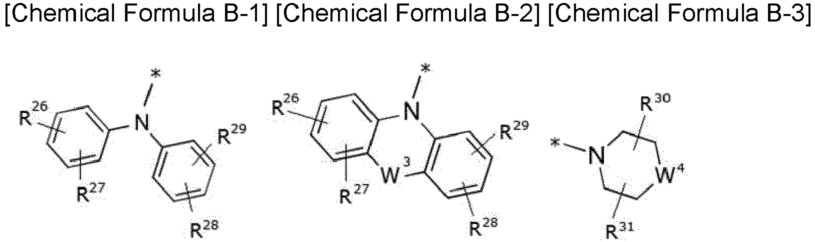

In Chemical Formulae B-1 to B-3,
W³ is a single bond, O, S, Se, NR^{j}, CR^{k}R^{l}, or SiR^{m}Rⁿ,
W⁴ is O, S, Se, NR^{j}, CR^{k}R^{l}, or SiR^{m}Rⁿ,
R²⁶ to R³¹ and R^{j} to Rⁿ are each independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof,
R²⁶ and R²⁷ may each be independently present or linked with each other to form a ring,
R²⁸ and R²⁹ may each be independently present or linked with each other to form a ring,
R³⁰ and R³¹ may each be independently present or linked with each other to form a ring,
R^{k} and R^{l} may each be independently present or linked with each other to form a ring,
R^{m} and Rⁿ may each be independently present or linked with each other to form a ring, and
* is a linking point.
in Chemical Formula B-1, Chemical Formula B-2, or Chemical Formula B-3, at least one hydrogen in the ring structure may be replaced by a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted silyl group, a halogen, or a combination thereof.

In some example embodiments, the compound may be represented by one of Chemical Formulae 1-G to 1-I depending on R¹ and R⁴, but is not limited thereto.

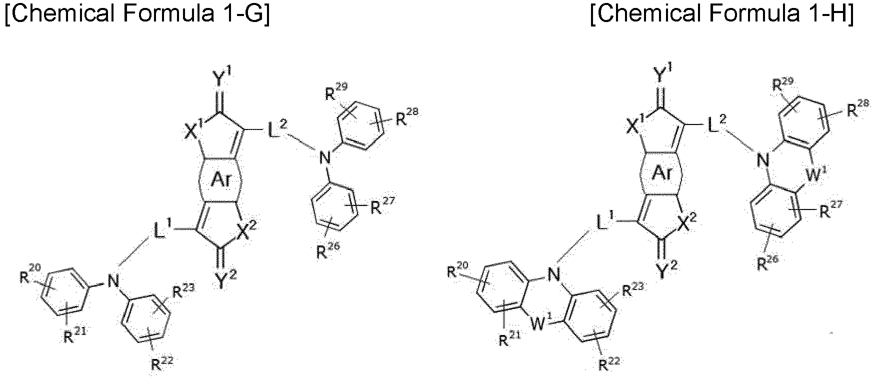

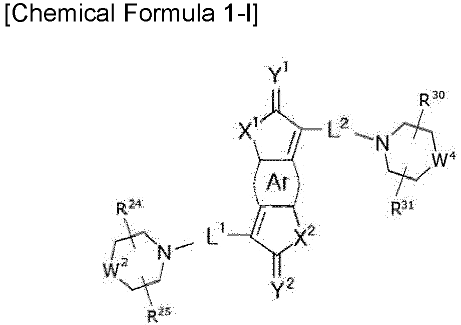

In Chemical Formula 1-G to 1-I, X¹, X², Y¹, Y², Ar, Z¹, Z², L¹, L², W¹ to W⁴, and R²⁰ to R³¹ are the same as described above.

In some example embodiments, the compound may be represented by Chemical Formulae 1-J to 1-Z2, but is not limited thereto.

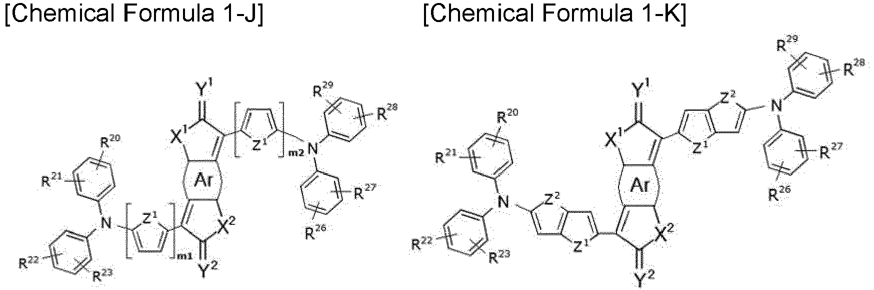

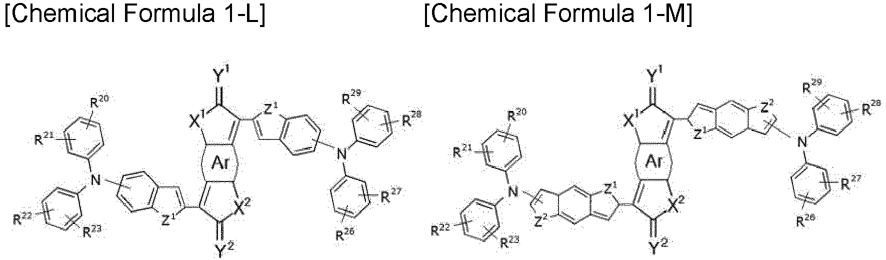

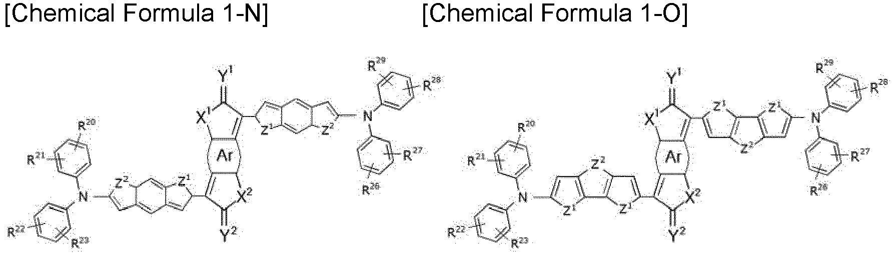

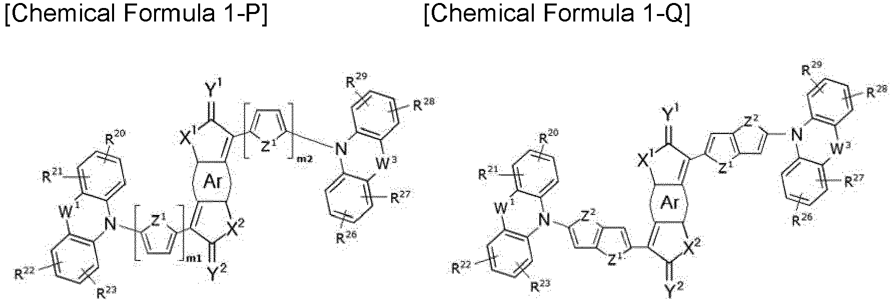

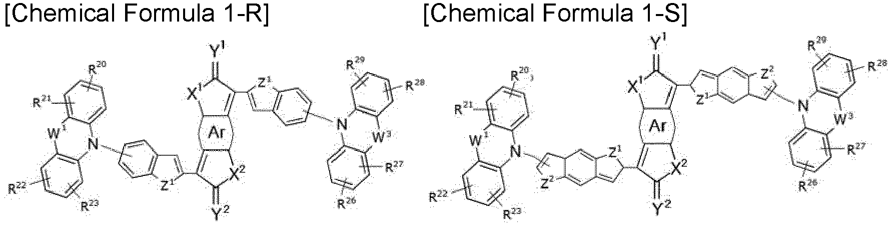

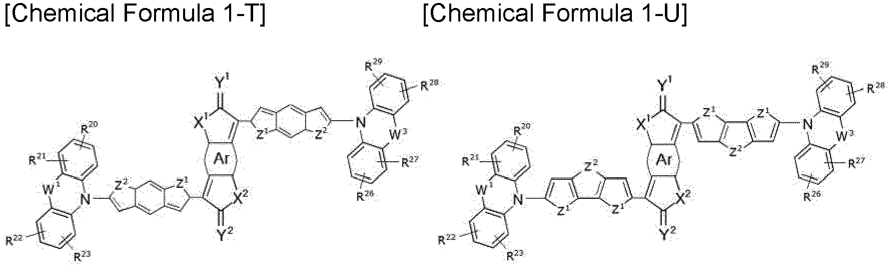

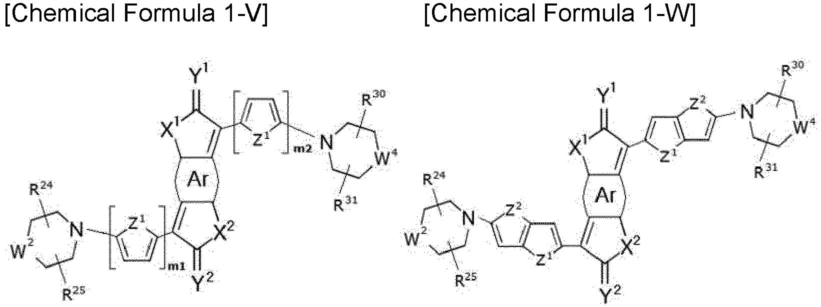

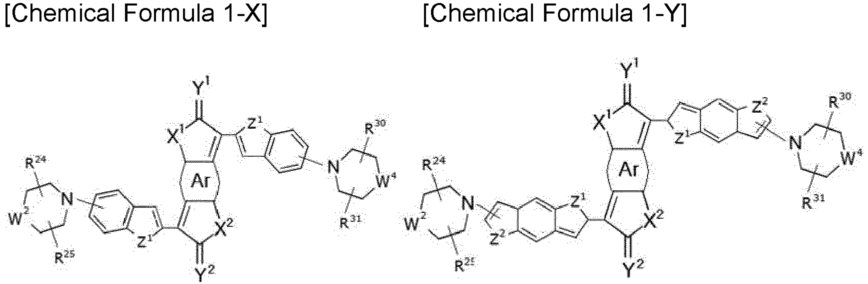

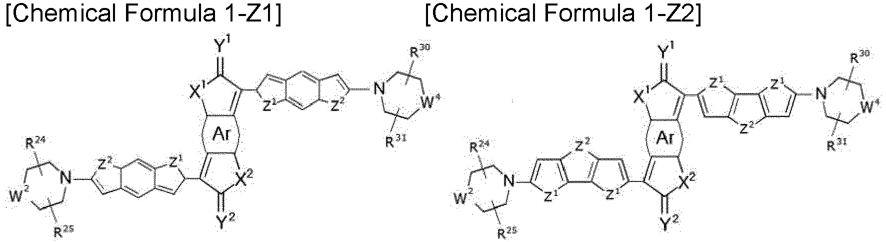

In Chemical Formulae 1-J to 1-Z2, X¹, X², Y¹, Y², Ar, Z¹, Z², W¹ to W⁴, R²⁰ to R³¹, m1, and m2 are the same as described above.

The compound may be a light absorbing material and may be, for example, configured to absorb light of a near infra-red wavelength spectrum. In some example embodiments, a peak absorption wavelength of the compound may be greater than or equal to 700 nm. In some example embodiments, the peak absorption wavelength of the compound may be within a wavelength spectrum of 700 nm to 3000 nm.

The compound may exhibit good charge transfer characteristics and accordingly has good photoelectric conversion characteristics for absorbing light and converting it into an electrical signal, so that the compound may be effectively used as a photoelectric conversion material of a photoelectric diode.

The compound has good heat resistance, which may prevent or reduce thermal decomposition during deposition, and thus may be deposited repeatedly. The compound may be thermally or vacuum deposited and may be deposited, for example, by sublimation. For example, deposition by sublimation may be confirmed by thermogravimetric analysis (TGA), and at a thermogravimetric analysis at a pressure of less than or equal to about 10 Pa, a temperature at which a 10% weight loss relative to an initial weight occurs may be less than or equal to about 450° C and a temperature at which a 50% weight loss relative to an initial weight occurs may be less than or equal to about 500° C.

In some example embodiments, at a thermogravimetric analysis of the compound at a pressure of less than or equal to about 10 Pa, for example temperature at which a 10% weight loss relative to an initial weight occurs may be about 230° C to about 450° C and a temperature at which a 50% weight loss relative to an initial weight occurs may be about 300° C to about 500° C.

The compound may be manufactured as a film.

The film may be applied to various fields requiring light absorption characteristics, for example a near infra-red absorption/cut film.

Since the compound has both light absorption characteristics and photoelectric characteristics, the compound may be effectively used as a photoelectric conversion material.

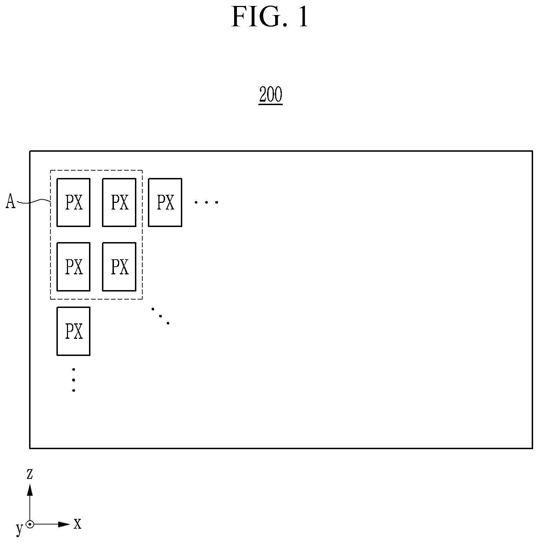

FIG. 1 is a schematic view showing an example of a pixel array of a sensor according to some example embodiments.

The organic sensor 200 according to some example embodiments includes a plurality of pixels (PX) and the plurality of pixels (PX) may have a matrix array repeatedly arranged along rows and columns. The plurality of pixels (PX) may form ("at least partially comprise") a unit pixel group (A) of for example a 2x2 array of pixels. However, an arrangement of the pixels are not limited thereto but variously modified, and the unit pixel group (A) may be variously modified into different arrays of pixels, including a 3x3 array, a 4x4 array, or the like, besides the 2x2 array.

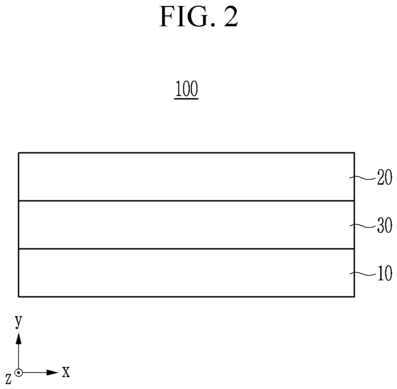

FIG. 2 is a cross-sectional view of a photoelectric diode according to some example embodiments.

Referring to FIG. 2, a photoelectric diode 100 according to some example embodiments includes a first electrode 10 and a second electrode 20 facing each other and an organic layer 30 between the first electrode 10 and the second electrode 20. A substrate (not shown) may be disposed at the side of the first electrode 10 or the second electrode 20. The substrate may be for example made of an inorganic material such as glass or a silicon wafer; an organic material such as polycarbonate, polymethylmethacrylate, polyethyleneterephthalate, polyethylenenaphthalate, polyamide, polyethersulfone, or any combination thereof; or an organic-inorganic hybrid material. The substrate may also be omitted.

One of the first electrode 10 and the second electrode 20 is an anode and the other is a cathode. In some example embodiments, the first electrode 10 may be an anode and the second electrode 20 may be a cathode.

At least one of the first electrode 10 and the second electrode 20 may be a light-transmitting electrode and the light-transmitting electrode may be for example made of a conductive oxide such as an indium tin oxide (ITO), indium zinc oxide (IZO), zinc oxide (ZnO), tin oxide (SnO), aluminum tin oxide (AlTO), and fluorine doped tin oxide (FTO), or a metal thin layer of a single layer or a multilayer. When one of the first electrode 10 and the second electrode 20 is a non-light-transmitting electrode, the non-light-transmitting electrode may be made of for example an opaque conductor such as aluminum (Al), silver (Ag), or gold (Au). In some example embodiments, the first electrode 10 and the second electrode 20 may be all light-transmitting electrodes. In some example embodiments, the second electrode 20 may be a light-receiving electrode disposed at a light receiving side.

The organic layer 30 may include an active layer.

The active layer is a layer including a p-type semiconductor and an n-type semiconductor to provide a PN junction, which is a layer producing excitons by receiving electromagnetic radiation and then separating holes and electrons from the produced excitons.

The p-type semiconductor and the n-type semiconductor may be independently a light-absorbing material configured to absorb light in at least one part of a wavelength spectrum and the aforementioned compound may be a p-type semiconductor or an n-type semiconductor. In some example embodiments, the aforementioned compound may be used for a p-type semiconductor and fullerene or a fullerene derivative may be included as an n-type semiconductor.

The active layer may include an intrinsic layer in which the aforementioned p-type semiconductor and an n-type semiconductor including fullerene derivative may be co-deposited. Herein, the p-type semiconductor and the n-type semiconductor may be included in a relative thickness ratio (volume ratio) of about 1:9 to about 9:1, for example about 2:8 to about 8:2, about 3:7 to about 7:3, about 4:6 to about 6:4, or about 5:5.

The active layer may further include a p-type layer and/or an n-type layer in addition to the intrinsic layer. The p-type layer may include the aforementioned p-type semiconductor and the n-type layer may include the aforementioned n-type semiconductor. In some example embodiments, the active layer may be included in various combinations of p-type layer/I layer, I layer/n-type layer, p-type layer/I layer/n-type layer, and the like.

The organic layer 30 may effectively absorb light in a wavelength spectrum of incident light (e.g., a near infra-red wavelength spectrum) and may photoelectrically convert it (i.e. the absorbed wavelength spectrum of light) into electric signals by including the above-described compound. Restated, the organic layer 30 may be or include a film that includes the composition according to any of the example embodiments described herein, such that the organic layer 30 includes the compound according to any of the example embodiments as described herein. For example, in some example embodiments, a peak absorption wavelength of the organic layer 30 may be greater than or equal to about 700 nm.

The peak absorption wavelength of the organic layer 30 may be within a wavelength spectrum of, for example about 700 nm to about 3000 nm, about 750 nm to about 2500 nm, about 780 nm to about 2200 nm, about 790 nm to about 2100 nm, about 800 nm to about 2000 nm, about 810 nm to about 2000 nm, about 820 nm to about 2000 nm, about 830 nm to about 2000 nm, about 850 nm to about 1900 nm, about 870 nm to about 1800 nm, about 900 nm to about 1600 nm, or about 910 nm to about 1500 nm.

The organic layer 30 may further include a charge auxiliary layer (not shown) between the first electrode 10 and the active layer and/or the second electrode 20 and the active layer. The charge auxiliary layer may allow the holes and electrons separated in the active layer to be transported more easily and could improve efficiency.

The charge auxiliary layer may include at least one of a hole injection layer (HIL) for facilitating hole injection, a hole transport layer (HTL) for facilitating hole transport, an electron blocking layer (EBL) for preventing electron transport, an electron injection layer (EIL) for facilitating electron injection, an electron transport layer (ETL) for facilitating electron transport, and a hole blocking layer (HBL) for preventing hole transport.

The charge auxiliary layer may include for example an organic material, an inorganic material, or an organic/inorganic material. The organic material may be an organic material having hole or electron injection, transport, or blocking characteristics and the inorganic material may be for example a metal oxide such as a molybdenum oxide, a tungsten oxide, or a nickel oxide.

The charge auxiliary layer may include for example the aforementioned compound.

The photoelectric diode 100 may further include an anti-reflection layer (not shown) on the first electrode 10 or the second electrode 20. The anti-reflection layer is disposed at a light incidence side and may lower reflectance of light of incident light and thereby light absorbance may be further improved. In some example embodiments, when light enters into the upper surface of the first electrode 10, the anti-reflection layer may be disposed on the upper surface of the first electrode 10, while when light enters into the lower surface of the second electrode 20, the anti-reflection layer may be disposed under the lower surface of the second electrode 20.

The anti-reflection layer may include, for example a material having a refractive index of about 1.6 to about 2.5 and may include for example at least one of a metal oxide, a metal sulfide, and an organic material having a refractive index within the ranges. The anti-reflection layer may include, for example a metal oxide such as an aluminum-containing oxide, a molybdenum-containing oxide, a tungsten-containing oxide, a vanadium-containing oxide, a rhenium-containing oxide, a niobium-containing oxide, a tantalum-containing oxide, a titanium-containing oxide, a nickel-containing oxide, a copper-containing oxide, a cobalt-containing oxide, a manganese-containing oxide, a chromium-containing oxide, a tellurium-containing oxide, or a combination thereof; a metal sulfide such as zinc sulfide; or an organic material such as an amine derivative, but is not limited thereto.

In the photoelectric diode 100, when light enters from the first electrode 10 or the second electrode 20 and the organic layer 30 absorbs light in a predetermined wavelength spectrum, excitons may be generated thereinside. The excitons are separated into holes and electrons in the organic layer 30, and the separated holes are transported to an anode that is one of the first electrode 10 and the second electrode 20 and the separated electrons are transported to the cathode that is the other of the first electrode 10 and the second electrode 20 so as to flow a current.

The photoelectric diode 100 may be applied to a solar cell, an image sensor, a photodetector, a photosensor, and a light emitting diode (LED), but is not limited thereto.

The photoelectric diode may be for example applied to an organic sensor. The organic sensor may be an organic CMOS sensor, for example an organic CMOS infrared light sensor or an organic CMOS image sensor.

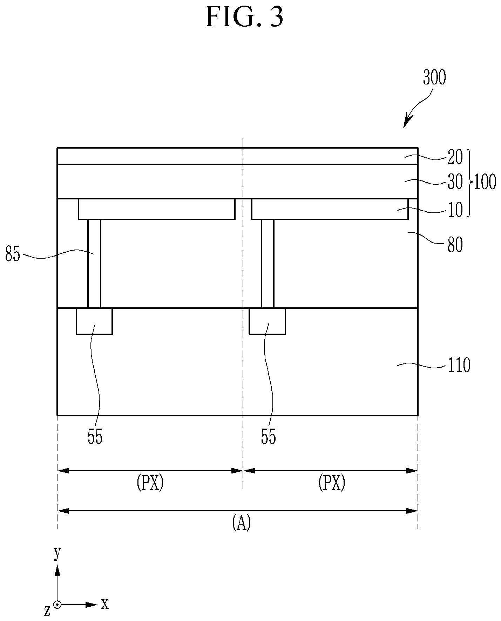

FIG. 3 is a cross-sectional view showing an organic sensor according to some example embodiments.

The organic sensor 300 according to some example embodiments includes a semiconductor substrate 110, an insulation layer 80, and a photoelectric diode 100.

The semiconductor substrate 110 may be a silicon substrate and is integrated with a transmission transistor (not shown) and a charge storage 55. The charge storage 55 may be integrated in each pixel. The charge storage 55 is electrically connected to the photoelectric diode 100 that will be described later and information of the charge storage 55 may be transferred by the transmission transistor.

A wire (not shown) and a pad (not shown) are formed on the semiconductor substrate 110. In order to decrease signal delay, the wire and pad may be made of a material having low resistivity, in some example embodiments, aluminum (Al), copper (Cu), silver (Ag), and alloys thereof, but are not limited thereto. Further, it is not limited to the structure, and the wire and pad may be disposed under the semiconductor substrate 110.

The insulation layer 80 may be formed on the wire and pad. The insulation layer 80 may be made of an inorganic insulating material such as a silicon oxide and/or a silicon nitride, or a low dielectric constant (low K) material such as SiC, SiCOH, SiCO, and SiOF. The insulation layer 80 has a trench 85 exposing the charge storage 55. The trench 85 may be filled with fillers.

The photoelectric diode 100 may be formed on the insulation layer 80. As described above, the photoelectric diode 100 includes a first electrode 10, an organic layer 30, and a second electrode 20. Even though a structure in which the first electrode 10, the organic layer 30 and the second electrode 20 are sequentially stacked is shown as an example in the drawing, the present disclosure is not limited to this structure, and the second electrode 20, the organic layer 30, and the first electrode 10 may be arranged in this order.

The first electrode 10 and the second electrode 20 may both be transparent electrodes, and the organic layer 30 is the same as described above. The organic layer 30 may selectively absorb light in a near infra-red wavelength spectrum. Incident light from the side of the second electrode 20 may be photoelectrically converted by mainly absorbing light in a near infra-red wavelength spectrum in the organic layer 30.

Focusing lens (not shown) may be further formed on the photoelectric diode 100. The focusing lens may control a direction of incident light and gather the light in one region. The focusing lens may have a shape of, in some example embodiments, a cylinder or a hemisphere, but is not limited thereto.

The organic sensor according to the present embodiment may be an organic infrared light sensor, in some example embodiments an iris sensor or a depth sensor.

The iris sensor identifies a person by using unique iris characteristics of every person and specifically, taking an image of an eye of a user within an appropriate distance, processing the image, and comparing it with his/her stored image.

The depth sensor identifies a shape and a location of an object from its three-dimensional information by taking an image of the object within an appropriate distance from a user and processing the image. This depth sensor may be for example used as a face recognition sensor.

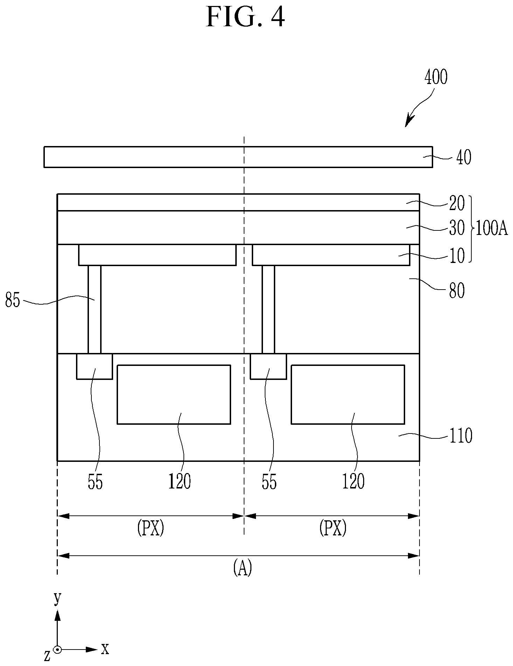

FIG. 4 is a cross-sectional view showing an organic sensor according to another embodiment.

The organic sensor according to the present embodiment may include a plurality of sensors having different functions. In some example embodiments, at least one of the plurality of sensors having different functions may be a biometric sensor, and the biometric sensor may be for example an iris sensor, a depth sensor, a fingerprint sensor, a blood vessel distribution sensor, and the like, but is not limited thereto. In some example embodiments, one of the plurality of sensors having different functions may be an iris sensor and the other may be a depth sensor.

In some example embodiments, a plurality of sensors may include, for example a first infrared light sensor configured to sense light in an infrared spectrum having a first wavelength (λ₁) in an infrared wavelength spectrum and a second infrared light sensor configured to sense light in an infrared spectrum having a second wavelength (λ₂) in an infrared wavelength spectrum.

The first wavelength (λ₁) and the second wavelength (λ₂) may be for example different in a wavelength spectrum of about 750 nm to about 3000 nm, and for example a difference between the first wavelength (λ₁) and the second wavelength (λ₂) may be greater than or equal to about 30 nm, greater than or equal to about 50 nm, greater than or equal to about 70 nm, greater than or equal to about 80 nm, or greater than or equal to about 90 nm.

In some example embodiments, one of the first wavelength (λ₁) and the second wavelength (λ₂) may be within a wavelength spectrum of about 780 nm to about 900 nm and the other of the first wavelength (λ₁) and the second wavelength (λ₂) may be within a wavelength spectrum of about 830 nm to about 1000 nm.

In some example embodiments, one of the first wavelength (λ₁) and the second wavelength (λ₂) may be within a wavelength spectrum of about 780 nm to about 840 nm and the other of the first wavelength (λ₁) and the second wavelength (λ₂) may be within a wavelength spectrum of about 910 nm to about 970 nm.

The organic sensor 400 according to the present embodiment includes a dual bandpass filter 40, a first infrared light sensor 100A, an insulation layer 80, and a semiconductor substrate 110 integrated with a second infrared light sensor 120. The first infrared light sensor 100A and the second infrared light sensor 120 may be stacked.

The dual bandpass filter 40 may be disposed on a front side of the organic sensor 400 and may selectively transmit infrared light including the first wavelength (λ₁) and infrared light including the second wavelength (λ₂) and may block and/or absorb other light. Herein, other light may include light in an ultraviolet (UV) and visible spectrum.

The first infrared light sensor 100A may be the photoelectric diode 100 according to the aforementioned embodiment and details thereof are omitted.

The second infrared light sensor 120 may be integrated in the semiconductor substrate 110 and may be a photo-sensing device. The semiconductor substrate 110 may be for example a silicon substrate and may be integrated with the second infrared light sensor 120, the charge storage 55, and a transmission transistor (not shown).

The second infrared light sensor 120 may be a photodiode and may sense entering light, and the sensed information may be transferred by the transmission transistor. Herein, the light entered into the second infrared light sensor 120 is light that passes the dual bandpass filter 40 and the first infrared light sensor 100A and may be infrared light in a predetermined spectrum including the second wavelength (λ₂). In a case where all infrared light in a predetermined spectrum including the first wavelength (λ₁) may be absorbed by the organic layer 30 and may not reach the second infrared light sensor 120, a separate filter having a wavelength selectivity with respect to the light entered into the second infrared light sensor 120 is not separately needed. However, for the case when all infrared light in a predetermined spectrum including the first wavelength (λ₁) is not absorbed by organic layer 30, a filter may be further disposed between the first infrared light sensor 100A and the second infrared light sensor 120.

The organic sensor according to the present embodiment may include two infrared light sensors respectively performing separate functions and thus may work as a combination sensor. In addition, two sensors performing separately functions are stacked in each pixel, and thus the number of pixel performing functioning of each sensor is twice increased while maintaining a size and resultantly, sensitivity may be much improved.

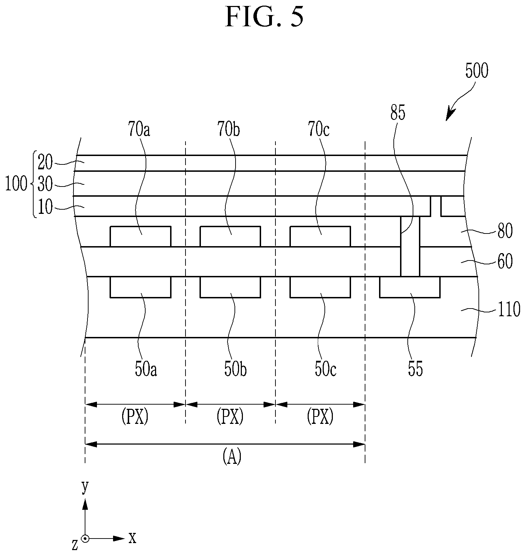

FIG. 5 is a cross-sectional view showing an example of an organic sensor according to some example embodiments.

An organic sensor according to some example embodiments may be an organic CMOS image sensor.

Referring to FIG. 5, an organic sensor 500 according to some example embodiment includes a semiconductor substrate 110 integrated with photo-sensing devices 50a, 50b, and 50c, a transmission transistor (not shown), and a charge storage 55, a lower insulation layer 60, color filter layers 70a, 70b, and 70c, an upper insulation layer 80, and a photoelectric diode 100.

The semiconductor substrate 110 may be integrated with photo-sensing devices 50a, 50b, and 50c, a transmission transistor (not shown), and a charge storage 55. The photo-sensing devices 50a, 50b, and 50c may be photodiodes.

The photo-sensing devices 50a, 50b, and 50c, the transmission transistor, and/or the charge storage 55 may be integrated in each pixel (PX). For example, the photo-sensing device 50a may be included in a red pixel, the photo-sensing device 50b may be included in a green pixel, and the photo-sensing device 50c may be included in a blue pixel.

The photo-sensing devices 50a, 50b, and 50c sense light, the information sensed by the photo-sensing devices may be transferred by the transmission transistor, the charge storage 55 is electrically connected to the photoelectric diode 100 that will be described later, and the information of the charge storage 55 may be transferred by the transmission transistor.

A wire (not shown) and a pad (not shown) are formed on the semiconductor substrate 110. In order to decrease signal delay, the wire and pad may be made of a material having low resistivity, for example, aluminum (Al), copper (Cu), silver (Ag), and alloys thereof, but are not limited thereto. Further, it is not limited to the structure, and the wire and pad may be disposed under the photo-sensing devices 50a and 50b.

The lower insulation layer 60 may be formed on the wire and the pad.

Color filters 70a, 70b, and 70c are formed on the lower insulation layer 60. The color filters 70a, 70b, and 70c includes a red filter 70a formed in a red pixel, a green filter 70b formed in a green pixel, and a blue filter 70c formed in a blue pixel.

The upper insulation layer 80 may be formed on the color filters 70a, 70b, and 70c. The upper insulation layer 80 eliminates steps caused by the color filters 70a, 70b, and 70c and planarizes the surface.

The photoelectric diode 100 may be formed on the upper insulation layer 80. As described above, the photoelectric diode 100 includes a first electrode 10, an organic layer 30, and a second electrode 20. Even though a structure in which the first electrode 10, the organic layer 30 and the second electrode 20 are sequentially stacked is shown as an example in the drawing, the present disclosure is not limited to this structure, and the second electrode 20, the organic layer 30, and the first electrode 10 may be arranged in this order.

The first electrode 10 and the second electrode 20 may both be transparent electrodes, and the organic layer 30 is the same as described above. The organic layer 30 may selectively absorb light in a near infra-red wavelength spectrum.

Incident light from the side of the second electrode 20 may be photoelectrically converted by mainly absorbing light in a near infra-red wavelength spectrum in the organic layer 30. Light in the remaining wavelength spectrum may pass through the first electrode 10 and the color filters 70a, 70b, and 70c, the light in a red wavelength spectrum passing through the color filter 70a may be sensed by the photo-sensing device 50a, the light in a green wavelength spectrum passing through the color filter 70b may be sensed by the photo-sensing device 50b, and the light in a blue wavelength spectrum passing through the color filter 70c may be sensed by the photo-sensing device 50c.

The organic sensor may be applied to various electronic devices, for example, the electronic devices may include for example a camera, a camcorder, a mobile phone, a display device, a security device, or a medical device internally having them, but are not limited thereto.

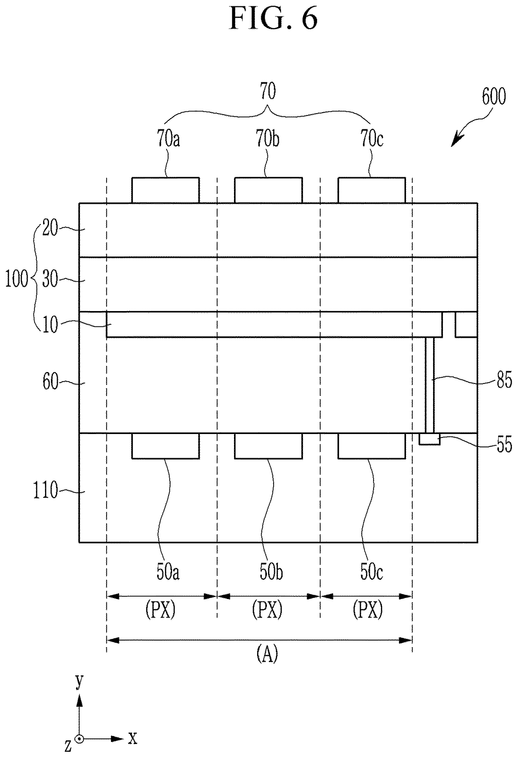

FIG. 6 is a schematic cross-sectional view of an organic sensor according to some example embodiments.

Referring to FIG. 6, an organic sensor 600 according to some example embodiments includes a semiconductor substrate 110 integrated with photo-sensing devices 50a, 50b, and 50c, a transmission transistor (not shown) and a charge storage 55, a lower insulation layer 60, a color filter layer 70 including color filters 70a, 70b, and 70c, and a photoelectric device 100. As shown in FIG. 6, the photoelectric device 100 may be between the semiconductor substrate 110 and the color filter layer 70, such that the color filter layer 70 is distal from the photo-sensing devices 50a, 50b, and 50c in relation to the photoelectric device 100. Other structures are the same as the organic sensor of FIG. 5.

In some example embodiments, the color filter layer 70 may include color filters configured to filter a mixture of wavelength spectra of light (e.g., mixed colors). For example, in FIG. 5 or 6, color filter 70a may be configured to filter magenta light, color filter 70b may be configured to filter cyan light, and color filter 70b may be configured to filter yellow light, while photo-sensing device 50a may be configured to detect ("sense") blue light and photo-sensing device 50b may be configured to detect red light.

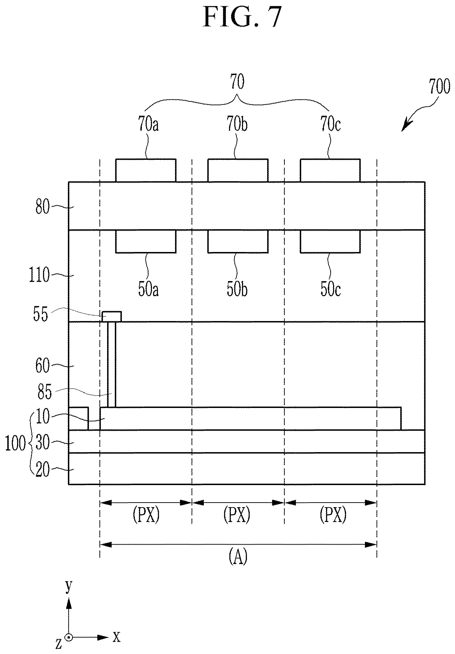

FIG. 7 is a schematic cross-sectional view of an organic sensor according to some example embodiments.

Referring to FIG. 7, an organic image sensor 700 according to some example embodiments includes a semiconductor substrate 110 integrated with photo-sensing devices 50a, 50b, and 50c, a transmission transistor (not shown) and a charge storage 55, a lower insulation layer 60, a color filter layer 70 and an upper insulation layer 80 on the semiconductor substrate 110, and a photoelectric device 100 under the semiconductor substrate 110.

As shown in FIG. 7, the photoelectric device 100 may be on (e.g., above or beneath) the semiconductor substrate 110, such that the color filter layer 70 is distal from the photoelectric device 100 in relation to the photo-sensing devices 50a, 50b, and 50c. Other structures are the same as the organic sensor of FIG. 5.

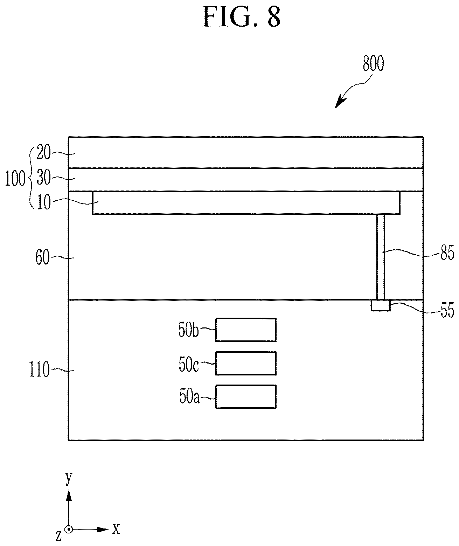

FIG. 8 is a cross-sectional view showing an organic sensor according to some example embodiments.

An organic sensor 800 according to some example embodiments includes a semiconductor substrate 110 integrated with photo-sensing devices 50a, 50b, and 50c, a transmission transistor (not shown), and a charge storage 55, an insulation layer 60 having a trench 85, and a photoelectric device 100. In the organic sensor 800 according to some example embodiments, the photo-sensing devices 50a, 50b, and 50c are stacked in a vertical direction and the color filter layer 70 is omitted. The photo-sensing devices 50a, 50b, and 50c are electrically connected to charge storage (not shown) and their information may be transferred by the transmission transistor. The photo-sensing devices 50a, 50b, and 50c may selectively absorb light in each wavelength spectrum of light depending on a stack depth. Other structures are the same as the organic sensor of FIG. 5.

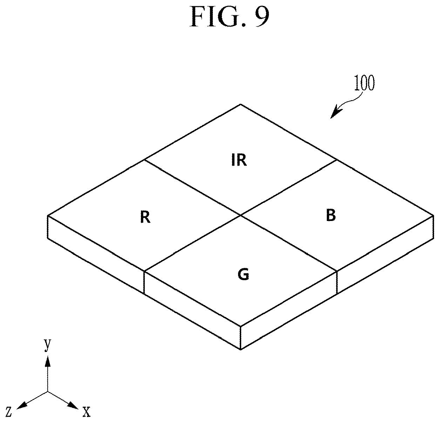

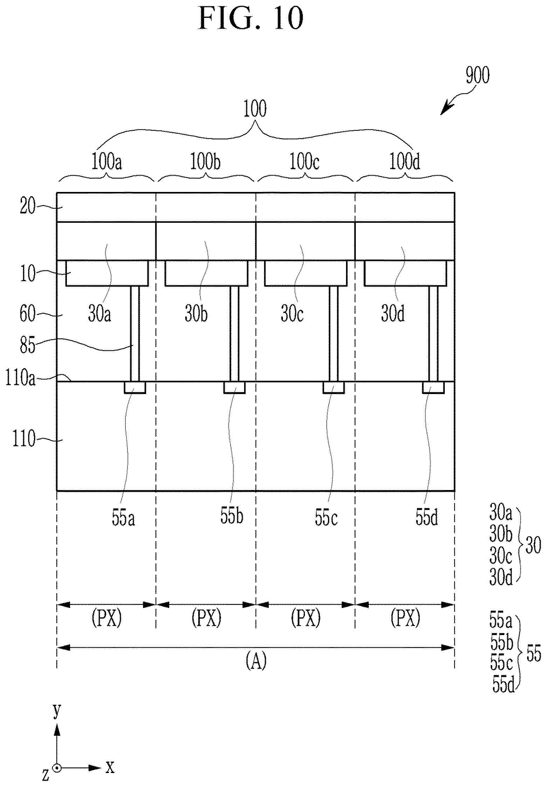

FIG. 9 is a perspective view of an organic sensor according to some example embodiments. FIG. 10 is a schematic cross-sectional view showing the organic sensor of FIG. 9, according to some example embodiments.

As shown in FIG. 9, organic sensor 900 according to some example embodiments includes an infrared/near infrared photoelectric device configured to selectively absorb light in an infrared/near infrared wavelength spectrum of light, a red photoelectric device configured to selectively absorb and convert (into electric signals) light in a red wavelength spectrum of incident light, a green photoelectric device configured to selectively absorb and convert (into electric signals) light in a green wavelength spectrum of incident light, and a blue photoelectric device configured to selectively absorb and convert (into electric signals) light in a blue wavelength spectrum of incident light, and they are horizontally arranged. As shown in FIG. 10, an organic sensor 900 may include a photoelectric device 100 that includes a plurality of photoelectric devices 100a, 100b, 100c, and 100d on a semiconductor substrate 110, where the plurality of photoelectric devices 100a, 100b, 100c, and 100d are configured to absorb and convert different ones of blue light, green light, red light, and infrared light (i.e., different wavelength spectra of incident light) into electric signals, respectively. Referring to FIG. 10, an organic CMOS image sensor 800 according to some example embodiments includes a semiconductor substrate 110 integrated with charge storages 55a to 55d, and transmission transistors (not shown), an insulation layer 60, and photoelectric devices 100a-100d. As shown in FIGS. 9-10, an organic sensor 900 may include a photoelectric device 100 that itself includes a plurality of photoelectric devices 100a-100d on a semiconductor substrate 110, where the plurality of photoelectric devices 100a-100d are configured to absorb and convert different ones of blue light, green light, red light, and infrared light (i.e., different wavelength spectra of incident light) into electric signals, respectively.

As shown in FIG. 10, the separate photoelectric devices 100a-100d may be horizontally arranged on the semiconductor substrate 110 such that the photoelectric devices 100a-100d are partially overlapped with each other in a direction that extends in parallel with a top surface 110a of the semiconductor substrate 110. As shown, each separate photoelectric device 100a to 100d is connected to a separate charge storage 55 that is integrated into the semiconductor substrate 110 via a separate trench 85. Each photoelectric device 100a to 100d may be any one of the photoelectric devices described herein. In some example embodiments, separate photoelectric devices 100a to 100d may include different portions of a common, continuous layer that extends continuously between two or more of the photoelectric devices 100a to 100d.

In some example embodiments, the photoelectric devices 100a to 100d may share a common opposed electrode 20. In another example, two or more of the photoelectric devices 100a to 100d may have different photoelectric conversion layers 30a, 30b, 30c, and 30d that are configured to absorb different wavelength spectra of incident light. Other structures are the same as the organic sensor of FIG. 4.

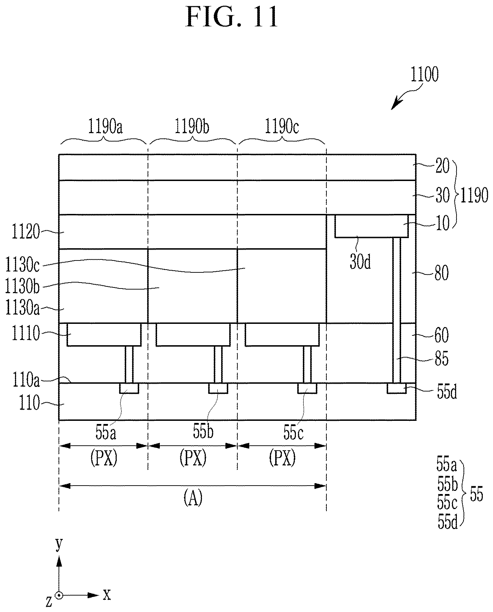

FIG. 11 is a schematic cross-sectional view of an organic sensor according to some example embodiments.

Referring to FIG. 11, an organic CMOS image sensor 1100 includes a semiconductor substrate 110 integrated with charge storages, and transmission transistors (not shown), an intermediate insulation layer 60, a first photoelectric device 1190, a second photoelectric device 1190a, a third photoelectric device 1190b, and a fourth photoelectric device 1190c. The first photoelectric device 1190, which may be an infrared/near infrared photoelectric device and thus may be configured to absorb infrared and/or near infrared light, may be formed on an entire surface of the second to fourth photoelectric devices 1190a to 1190c. The second to fourth photoelectric devices 1190a, 1190b, and 1190c may be configured to absorb and convert different ones of blue light, green light, and red light (i.e., different wavelength spectra of incident light), respectively. For example, as shown, the second to fourth photoelectric devices 1190a, 1190b, and 1190c may share a continuous opposed electrode 1120 and may include separate, respective pixel electrodes 1110 and may further each include separate, respective photoelectric conversion layers 1130a, 1130b, and 1130c that may be configured to absorb and convert separate, respective wavelength spectra of light (e.g., red light, green light, and blue light, respectively). Other structures are the same as the image sensor of FIG. 4.

Referring to FIG. 11, the first photoelectric device 1190 may be stacked on the second to fourth photoelectric device 1190a to 1190c so as to at least partially overlap them in a direction extending perpendicular to the top surface 110a of the semiconductor substrate 110, and wherein the second to fourth photoelectric devices 1190a to 1190c overlap in a direction extending parallel to the top surface 110a of the semiconductor substrate 110. It will be understood that, in some example embodiments, the second to fourth photoelectric devices 1190a to 1190c include multiple, horizontally-arranged photoelectric devices configured to absorb different wavelengths spectra of light while the first photoelectric device 1190 is limited to a single photoelectric device that is configured to absorb a single wavelength or a tailored spectrum of light.

In some example embodiments, including the example embodiments shown in FIG. 11, an entirety of the first photoelectric device 1190 overlaps a limited portion of the second to fourth photoelectric devices 1190a to 1190c in the direction extending perpendicular to the top surface 110a and a remainder portion of the first photoelectric device 1190 that is exposed by the second to fourth photoelectric devices 1190a to 1190c is covered by insulation layer 80. However, it will be understood that in some example embodiments an entirety of the first photoelectric device 1190 overlaps a limited portion of the second to fourth photoelectric devices 1190a to 1190c in the direction extending perpendicular to the top surface 110a.

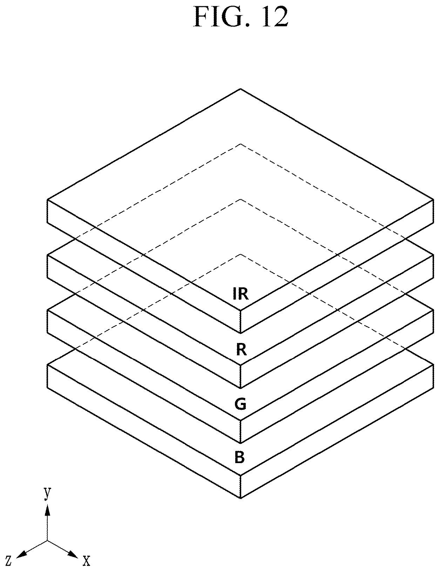

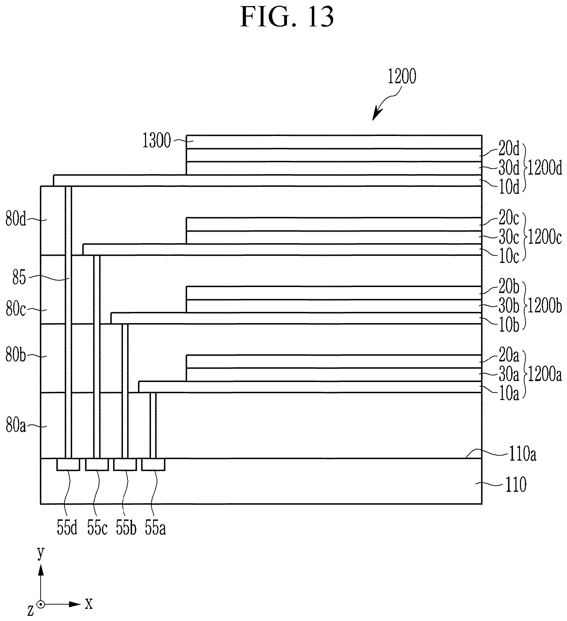

FIG. 12 is a perspective view of an organic sensor according to some example embodiments, and FIG. 13 is a schematic cross-sectional view showing the organic sensor of FIG. 12 according to some example embodiments.

As shown in FIG. 12, the organic sensor 1200 according to some example embodiments includes an infrared/near infrared photoelectric device configured to selectively absorb light in an infrared/near infrared wavelength spectrum of light, a red photoelectric device configured to selectively absorb and convert (into electric signals) light in a red wavelength spectrum of incident light, a green photoelectric device configured to selectively absorb and convert (into electric signals) light in a green wavelength spectrum of incident light, and a blue photoelectric device configured to selectively absorb and convert (into electric signals) light in a blue wavelength spectrum of incident light, and they are stacked. As shown in FIG. 13, the organic sensor 1200 according to some example embodiments includes a semiconductor substrate 110, a lower insulation layer 80a, an intermediate insulation layer 80b, another intermediate insulation layer 80c, an upper insulation layer 80d, a first photoelectric device 1200a, a second photoelectric device 1200b, a third photoelectric device 1200c, and a fourth photoelectric device 1200d. As shown, the first to fourth photoelectric devices 1200a to 1200d are stacked vertically on the semiconductor substrate 110, such that the first to fourth photoelectric devices 1200a to 1200d overlap each other in a direction perpendicular to a top surface 110a of the semiconductor substrate 110.

The semiconductor substrate 110 may be a silicon substrate, and is integrated with the transmission transistor (not shown) and the charge storages 55a, 55b, 55c, and 55d. The first photoelectric device 1200a may be formed on the lower insulation layer 80a.

The first photoelectric device 1200a includes a first electrode 10a and a second electrode 20a facing each other and a photoelectric conversion layer 30a between the first electrode 10a and the second electrode 20a. The first electrode 10a, the second electrode 20a, and the photoelectric conversion layer 30a are the same as described above and the photoelectric conversion layer 30a may selectively absorb and convert (into electric signals) light in one of infrared, red, blue, and green wavelength spectra of incident light. For example, the first photoelectric device 1200a may be a blue photoelectric device. In the drawing, the first electrode 10a, the photoelectric conversion layer 30a, and the second electrode 20a are sequentially stacked, but this disclosure is not limited thereto, and for example they may be stacked in an order of the second electrode 20a, the photoelectric conversion layer 30a, and the first electrode 10a.

An intermediate insulation layer 80b may be formed on the first photoelectric device 1200a. The second photoelectric device 1200b may be formed on the intermediate insulation layer 80b. The second photoelectric device 1200b may include a first electrode 10b and a second electrode 20b facing each other and a light-absorption layer 30b (e.g., photoelectric conversion layer 30b) between the first electrode 10b and the second electrode 20b. The first electrode 10b, the second electrode 20b, and the photoelectric conversion layer 30b are the same as described above and the photoelectric conversion layer 30b may selectively absorb and convert (into electric signals) light in one of infrared, red, blue, and green wavelength spectra of incident light. For example, the second photoelectric device 1200b may be a green photoelectric device. In the drawing, the first electrode 10b, the photoelectric conversion layer 30b, and the second electrode 20b are sequentially stacked, but this disclosure is not limited thereto, and for example they may be stacked in an order of the second electrode 20b, the photoelectric conversion layer 30b, and the first electrode 10b.

Another intermediate insulation layer 80c may be formed on the second photoelectric device 1200b. The third photoelectric device 1200c may be formed on the intermediate insulation layer 80c. The third photoelectric device 1200c includes a first electrode 10c and a second electrode 20c facing each other and a light-absorption layer 30c (e.g., photoelectric conversion layer 30c) between the first electrode 10c and the second electrode 20c. The first electrode 10c, the second electrode 20c, and the photoelectric conversion layer 30c may be the same as described above and the photoelectric conversion layer 30c may selectively absorb and convert (into electric signals) light in one of infrared, red, blue, and green wavelength spectra of incident light. For example, the third photoelectric device 1200c may be a red photoelectric device. In the drawing, the first electrode 10c, the photoelectric conversion layer 30c, and the second electrode 20c are sequentially stacked, but this disclosure is not limited thereto, and for example they may be stacked in an order of the second electrode 20c, the photoelectric conversion layer 30c, and the first electrode 10c. The upper insulation layer 80d may be formed on the third photoelectric device 1200c. The lower insulation layer 80a, the intermediate insulation layers 80b and 80c, and the upper insulation layer 80d have a plurality of through-holes exposing the charge storages 55a, 55b, 55c, and 55d. The fourth photoelectric device 1200d may be formed on the upper insulation layer 80d. The fourth photoelectric device 1200d includes a first electrode 10d and a second electrode 20d facing each other and a light-absorption layer 30d (e.g., photoelectric conversion layer 30d) between the first electrode 10d and the second electrode 20d. The first electrode 10d, the second electrode 20d, and the photoelectric conversion layer 30d may be the same as described above and the photoelectric conversion layer 30d may selectively absorb and convert (into electric signals) light in one of infrared, red, blue, and green wavelength spectra of light. For example, the fourth photoelectric device 1200d may be an infrared/near infrared photoelectric device. In the drawing, the first electrode 10d, the photoelectric conversion layer 30d, and the second electrode 20d are sequentially stacked, but this disclosure is not limited thereto, and for example they may be stacked in an order of the second electrode 20d, the photoelectric conversion layer 30d, and the first electrode 10d.

Focusing lens 1300 may be further formed on the fourth photoelectric device 1200d. The focusing lens 1300 may control a direction of incident light and gather the light in one region. The focusing lens 1300 may have a shape of, for example, a cylinder or a hemisphere, but is not limited thereto.

In the drawing, the first photoelectric device 1200a, the second photoelectric device 1200b, the third photoelectric device 1200c, and the fourth photoelectric device 1200d are sequentially stacked, but the present disclosure is not limited thereto, and they may be stacked in various orders.

As described above, the first photoelectric device 1200a, the second photoelectric device 1200b, the third photoelectric device 1200c, and the fourth photoelectric device 1200d have a stack structure, and thus the size of an image sensor may be reduced to realize a down-sized image sensor.

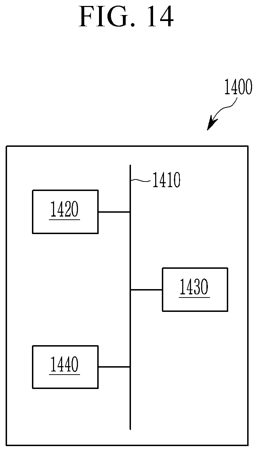

FIG. 14 is a schematic diagram of an electronic device according to some example embodiments.

As shown in FIG. 14, an electronic device 1400 may include a processor 1420, a memory 1430, and an organic sensor 1440 that are electrically coupled together via a bus 1410. The organic sensor 1440 may be an organic sensor of any of the example embodiments as described herein, and the organic sensor included in the organic sensor 1440 may include any of the photoelectric devices described herein according to any of the example embodiments of the inventive concepts. The memory 1430, which may be a non-transitory computer readable medium, may store a program of instructions. The processor 1420 may execute the stored program of instructions to perform one or more functions.

In some example embodiments, the processor 1420 may be configured to process electric signals generated by the organic sensor 1440. The processor 1420 may be configured to generate an output (e.g., an image to be displayed on a display interface) based on processing the electric signals. While some example embodiments of photoelectric devices shown herein include first and second electrodes 10 and 20 facing each other and an organic layer 30 therebetween, where the organic layer may include a compound according to any of the example embodiments herein, it will be understood that in some example embodiments at least the first and second electrodes 10 and 20 may be omitted from the photoelectric device.

Hereinafter, the embodiments are illustrated in more detail with reference to examples. However, these examples are exemplary, and the present scope is not limited thereto.

### Synthesis Examples

### Synthesis Example 1

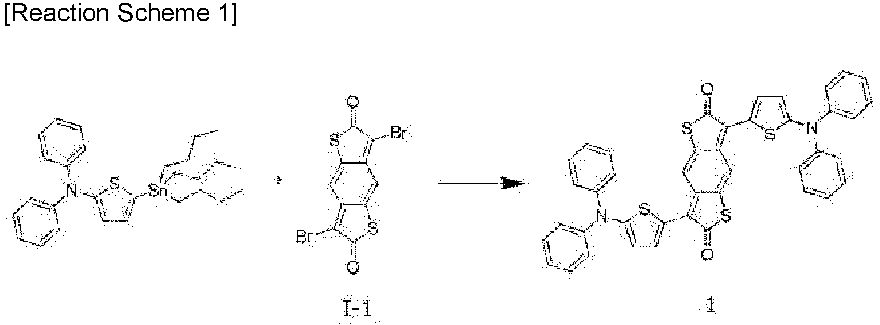

### Synthesis of Compound I-1

3,7-dibromobenzo[1,2-b:4,5-b']dithiophene-2,6-dione (Compound I-1) is synthesized according to the method described in Chem. Lett., 905 1983.

### Synthesis of Compound 1

3,7-dibromobenzo[1,2-b:4,5-b']dithiophene-2,6-dione (Compound I-1) (0.60 g, 1.59 mmol), N,N-diphenyl-5-(tributylstannyl)thiophen-2-amine (1.80 g, 3.33 mmol), and tetrakis(triphenylphosphine)palladium (0) (Pd(PPh₃)₄) (0.18 g, 0.16 mmol) are dissolved in 300 mL of toluene and then, stirred at 110° C for 18 hours. When a reaction is complete, the toluene is evaporated so that the mixture is concentrated and the resultant is separated with a dichloromethane:hexane (1:1 v/v) column and then, precipitated in 200 mL of methanol to obtain Compound 1 (0.61 g). A yield is 53%.

NMR (300 MHz, CDCl₃): 7.81 (d, 2H), 7.48 (s, 2H), 7.39 (t, 8H), 7.28 (d, 8H), 7.21 (t, 4H), 6.56 (d, 2H).

### Synthesis Example 2

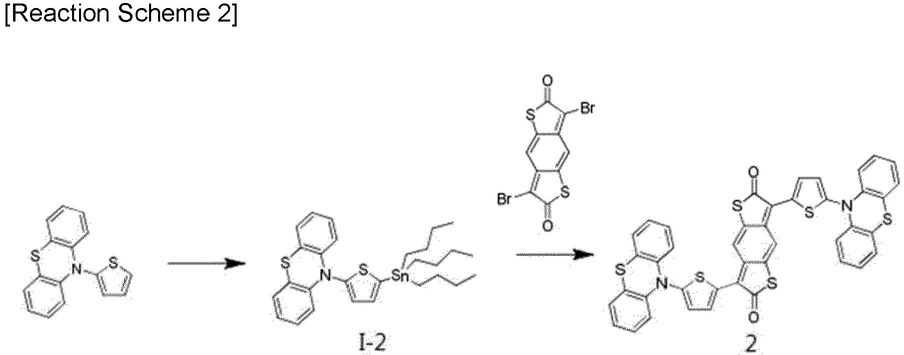

### Synthesis of Compound I-2

10-(thiophen-2-yl)-10H-phenothiazine (1.0 g, 3.55 mmol) is added to 50 mL of THF, 2.5 M n-BuLi (1.56 mL, 3.91 mmol) is slowly added thereto at -78° C and the mixture is stirred for 4 hours. Subsequently, tributyltin chloride (1.27 g, 3.91 mmol) is slowly added thereto at room temperature and the mixture is further stirred for 4 hours. When a reaction is complete, the resultant is extracted with 200 mL of chloroform to obtain 2 g of 10-(5-(tributylstannyl)thiophen-2-yl)-10H-phenothiazine (Compound I-2).

NMR (300 MHz, CD₂Cl₂): 7.25 (d, 1H), 7.22 (d, 1H), 7.05 (d, 2H), 6.99 (t, 2H), 6.90 (t, 2H), 6.67 (d, 2H), 1.65 (m, 6H), 1.40 (m, 6H), 1.20 (t, 6H), 0.94 (t, 9H).

### Synthesis of Compound 2

3,7-dibromobenzo[1,2-b:4,5-b']dithiophene-2,6-dione (Compound I-1) (0.30 g, 0.79 mmol), 10-(5-(tributylstannyl)thiophen-2-yl)-10H-phenothiazine (Compound I-2) (0.95 g, 1.67 mmol), and Pd(PPh₃)₄ (0.09 g, 0.08 mmol) are dissolved in 100 mL of toluene and then, stirred at 110° C for 18 hours. When a reaction is complete, the toluene is evaporated so that the mixture is concentrated, and the resultant is separated with a dichloromethane:hexane (1:1 v/v) column and then, precipitated in 100 mL of methanol to obtain Compound 2 (0.30 g). A yield is 49%.

NMR (300 MHz, CDCl₃): 7.80 (d, 2H), 7.53 (s, 2H), 7.33 (m, 8H), 7.21 (d, 4H), 7.15 (t, 4H), 6.82 (d, 2H).

### Synthesis Example 3

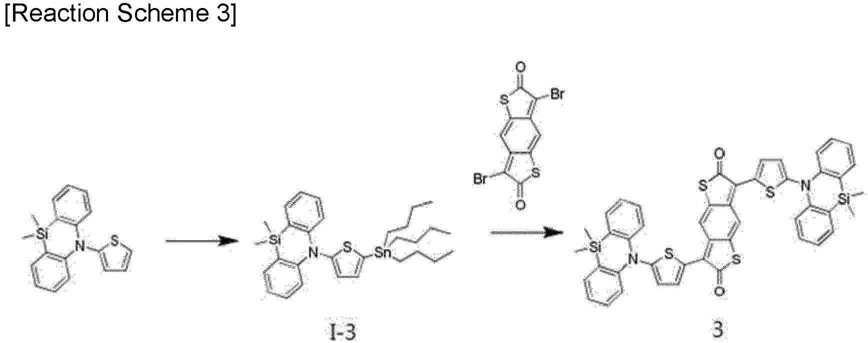

### Synthesis of Compound I-3

10,10-dimethyl-5-(thiophen-2-yl)-5,10-dihydrodibenzo[b,e][1,4]azasiline (1.0 g, 3.25 mmol) is added to 50 mL of THF and 2.5 M n-BuLi (1.43 mL, 3.58 mmol) is slowly added thereto at -78° C and the mixture is stirred for 4 hours. Subsequently, tributyltin chloride (1.06 g, 3.91 mmol) is slowly added thereto at room temperature and the mixture is further stirred for 4 hours. When a reaction is complete, the resultant is extracted with 200 mL of chloroform to obtain 1.9 g of 10,10-dimethyl-5-(5-(tributylstannyl)thiophen-2-yl)-5,10-dihydrodibenzo[b,e][1,4]azasiline (Compound I-3).

NMR (300 MHz, CD₂Cl₂) : 7.58 (d, 2H), 7.28 (d, 1H), 7.23 (t, 2H), 7.11 (d, 1H), 7.03 (t, 2H), 6.74 (d, 2H), 1.65 (m, 6H), 1.40 (m, 6H), 1.20 (t, 6H), 0.95 (t, 9H), 0.52 (s, 6H).

### Synthesis of Compound 3

3,7-dibromobenzo[1,2-b:4,5-b']dithiophene-2,6-dione (Compound I-1) (0.30 g, 0.79 mmol), 10,10-dimethyl-5-(5-(tributylstannyl)thiophen-2-yl)-5,10-dihydrodibenzo[b,e][1,4]azasiline (Compound I-3) (0.99 g, 1.67 mmol), and Pd(PPh₃)₄ (0.09 g, 0.08 mmol) are dissolved in 100 mL of toluene and then, stirred at 110° C for 18 hours. When a reaction is complete, the toluene is evaporated so that the mixture is concentrated and the resultant is separated with a dichloromethane:hexane (1:1 v/v) column and is precipitated in 100 mL of methanol to obtain Compound 3 (0.30 g). A yield is 45%.

Confirmation of molecular weight: LC-MS: 831.11 m/z.

### Synthesis Example 4

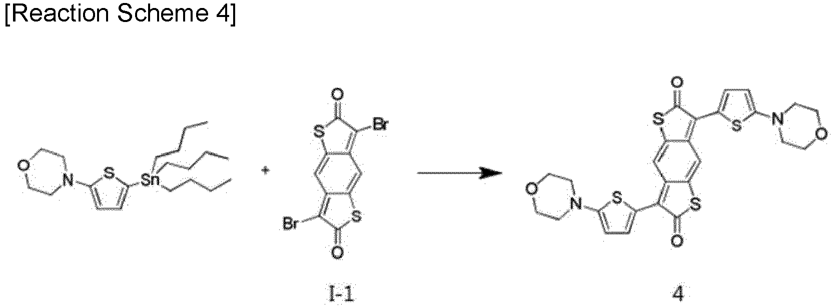

3,7-dibromobenzo[1,2-b:4,5-b']dithiophene-2,6-dione (Compound I-1) (0.30 g, 0.79 mmol), 4-(5-(tributylstannyl)thiophen-2-yl)morpholine (0.76 g, 1.67 mmol) and Pd(PPh₃)₄ (0.09 g, 0.08 mmol)are dissolved in 100 mL of toluene and then, stirred at 110 °C for 18 hours. When a reaction is complete, the toluene is evaporated so that the mixture is concentrated, and the resultant is separated with a dichloromethane:hexane (1:1 v/v) column and then, precipitated in in 100 mL of methanol to obtain Compound 4 (0.10 g). A yield is 22%.
confirmation of molecular weight: LC-MS : 555.07 m/z

### Synthesis Example 5

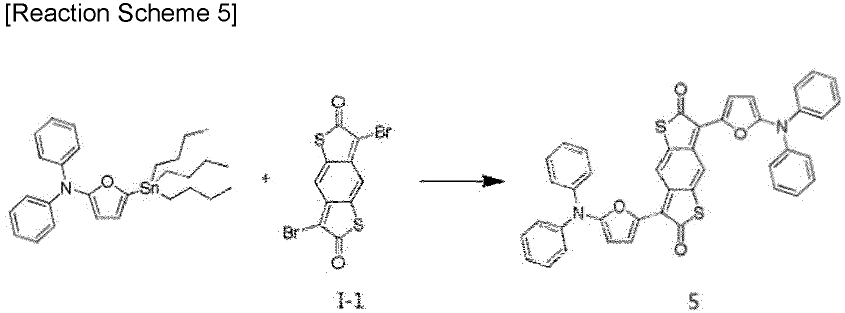

3,7-dibromobenzo[1,2-b:4,5-b']dithiophene-2,6-dione (Compound I-1) (0.30 g, 0.79 mmol), N,N-diphenyl-5-(tributylstannyl)furan-2-amine (0.87 g, 1.67 mmol), and Pd(PPh₃)₄ (0.09 g, 0.08 mmol) are dissolved in 100 mL of toluene and then, stirred at 110° C for 18 hours. When a reaction is complete, the toluene is evaporated so that the mixture is concentrated, and then, the resultant is separated with a dichloromethane:hexane (1:1 v/v) column and precipitated in 100 mL of methanol to obtain Compound 5 (0.25 g). A yield is 46%.

NMR (300 MHz, CDCl₃): 7.49 (d, 2H), 7.45 (s, 2H), 7.42 (d, 8H), 7.30 (d, 8H), 7.23 (t, 4H), 7.06 (d, 2H).

### Synthesis Example 6

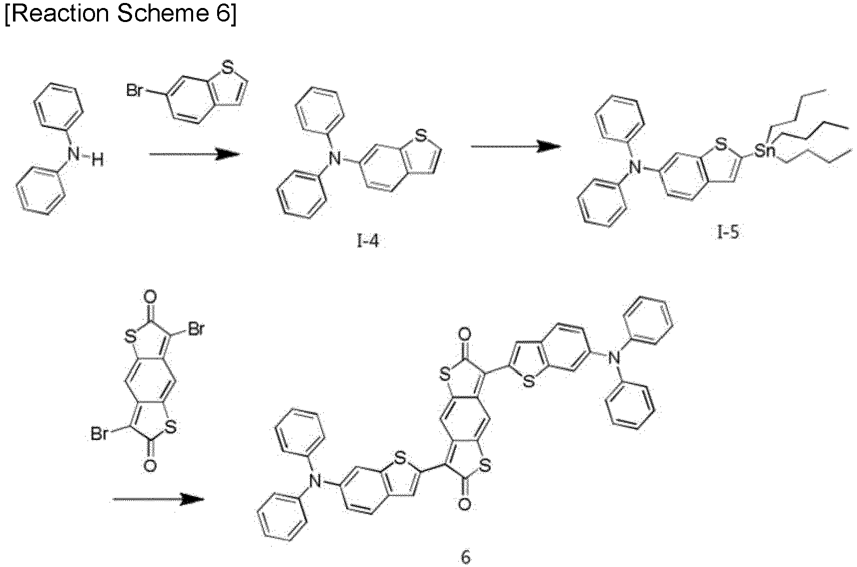

### Synthesis of Compound I-4

Diphenylamine (2 g, 11.82 mmol), 6-bromobenzo[b]thiophene (2.52 g, 11.82 mmol), 5 mol% Pd(dba)₂, 5 mol% P(tBu)₃, and NaOtBu (1.36 g, 14.18 mmol) are dissolved in 100 mL of toluene and then, stirred at 110° C for 18 hours. When a reaction is complete, the toluene is evaporated so that the mixture is concentrated, and the resultant is separated with a dichloromethane:hexane (1:1 v/v) column to obtain 2.5 g of N,N-diphenylbenzo[b]thiophen-6-amine (Compound I-4). A yield is 70%.

NMR (300 MHz, CDCl₃): 7.68 (d, 1H), 7.55 (s, 1H), 7.31 (d, 1H), 7.28 (d, 1H), 7.22 (d, 4H), 7.15 (d, 1H), 7.10 (d, 4H), 7.01 (t, 2H).

### Synthesis of Compound I-5

N,N-diphenylbenzo[b]thiophen-6-amine (Compound I-4) (2.0 g, 6.64 mmol) is added to 60 mL of THF, a 2.5 M n-BuLi (2.92 mL, 7.30 mmol) is slowly added thereto at -78°C and the mixture is stirred for 4 hours. Subsequently, tributyltin chloride (2.38g, 7.30 mmol) is slowly added thereto at room temperature and the mixture is further stirred for 4 hours. When a reaction is complete, the resultant is extracted with 200 mL of chloroform to obtain 3.9 g of N,N-diphenyl-2-(tributylstannyl)benzo[b]thiophen-6-amine (Compound I-5).

NMR (300 MHz, CDCl₃) : 7.67 (d, 1H), 7.57 (s, 1H), 7.31 (s, 1H), 7.22 (d, 4H), 7.13 (d, 1H), 7.10 (d, 4H), 6.99 (t, 2H), 1.62 (m, 6H), 1.38 (m, 6H), 1.14 (t, 6H), 0.90 (t, 9H).

### Synthesis of Compound 6

3,7-dibromobenzo[1,2-b:4,5-b']dithiophene-2,6-dione (Compound I-1) (0.20 g, 0.53 mmol), N,N-diphenyl-2-(tributylstannyl)benzo[b]thiophen-6-amine (Compound I-5) (0.66 g, 1.11 mmol), and Pd(PPh₃)₄ (0.06 g, 0.05 mmol) are dissolved in 60 mL of toluene and then, stirred at 110° C for 18 hours. When a reaction is complete, the toluene is evaporated so that the mixture is concentrated and the resultant is separated with a dichloromethane:hexane (1:1 v/v) column and is precipitated in 100 mL of methanol to obtain Compound 6 (0.2 g). A yield is 46%.

Confirmation of molecular weight: LC-MS: 819.11 m/z.

### Synthesis Example 7

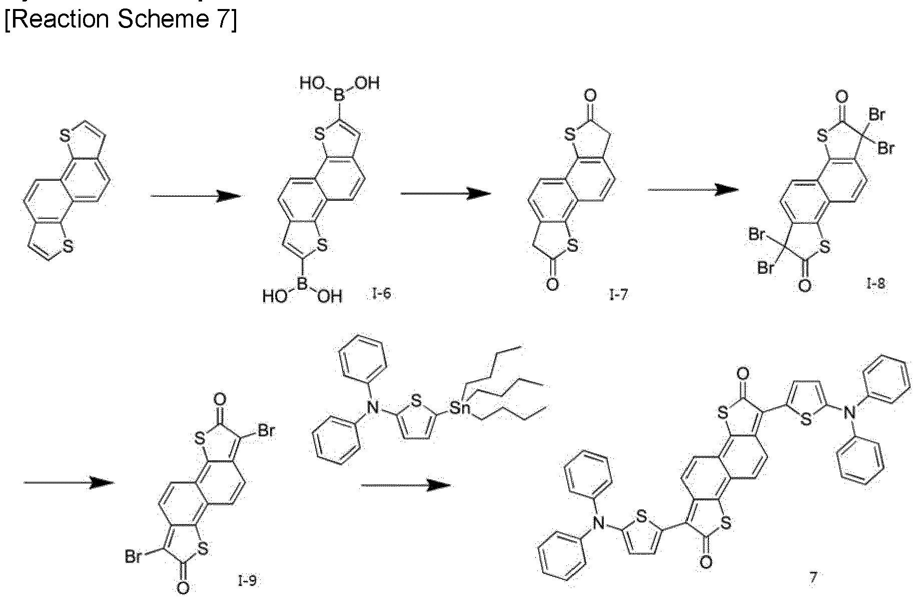

### Synthesis of Compound I-6

Naphtho[1,2-b:5,6-b']dithiophene (2 g, 8.32 mmol) is dissolved in a THF/ethyl ether (100mL/100 mL) mixed solvent, 2.5 M n-BuLi (8.32 mL, 20.80 mmol) is added at 0° C, and the mixture is stirred for 4 hours. Subsequently, triisopropyl borate (7.83 g, 41.61 mmol) is slowly added thereto at room temperature and the mixture is further stirred for 4 hours. Then, 1 M HCl is slowly added thereto, the solvent is evaporated so that the mixture is concentrated, and the resultant is added to 300 mL of H₂O to precipitate and filter it to obtain 2.7 g of naphtho[1,2-b:5,6-b']dithiophene-2,7-diyldiboronic acid (Compound I-6).

NMR (300 MHz, DMSO): 8.57 (s, 4H), 8.13 (s, 2H), 8.07 (s, 4H).

### Synthesis of Compound I-7

Naphtho[1,2-b:5,6-b']dithiophene-2,7-diyldiboronic acid (Compound I-6) (2.7g, 8.23 mmol) and oxone (6.56 g, 21.35 mmol) are dissolved in 150 mL of acetone and 40 mL of H₂O and stirred at room temperature for 48 hours. When precipitation occurs, it is filtered and washed with acetone and H₂O to obtain 1.7 g of naphtho[1,2-b:5,6-b']dithiophene-2,7(1H,6H)-dione (I-7). A yield is 76%.

NMR (300 MHz, CDCl₃) : 7.54 (d, 2H), 7.51 (d, 2H), 4.24 (s, 4H).

### Synthesis of Compound I-8

Naphtho[1,2-b:5,6-b']dithiophene-2,7(1H,6H)-dione (Compound I-7) (0.5 g, 1.84 mmol), N-bromosuccinimide (NBS) (1.63 g, 9.18 mmol), and azobisisobutyronitrile(AIBN) (0.03 g, 0.18 mmol) are dissolved in 150 mL of chloroform and stirred at 85° C for 18 hours. When a reaction is complete, the resultant is precipitated in 150 mL of MeOH to obtain 0.65 g of 1,1,6,6-tetrabromonaphtho[1,2-b:5,6-b']dithiophene-2,7(1H,6H)-dione (Compound I-8). A yield is 60%.

NMR (300 MHz, CDCl₃): 7.98 (d, 2H), 7.66 (d, 2H).

### Synthesis of Compound I-9

1,1,6,6-tetrabromonaphtho[1,2-b:5,6-b']dithiophene-2,7(1H,6H)-dione (Compound I-8) (0.43 g, 0.73 mmol), and sodium iodide (0.33 g, 2.19 mmol) are dissolved in 20 mL of acetonitrile and 40 mL of chloroform and stirred at room temperature for 18 hours. When a reaction is complete, the resultant is precipitated in 100 mL of acetone to obtain 0.15 g of 1,6-dibromonaphtho[1,2-b:5,6-b']dithiophene-2,7-dione (Compound I-9). A yield is 48%.

Confirmation of molecular weight: MALDI-TOF-MS: 428.95 m/z.

### Synthesis of Compound 7

1,6-dibromonaphtho[1,2-b:5,6-b']dithiophene-2,7-dione (Compound I-9) (0.15 g, 0.35 mmol), N,N-diphenyl-5-(tributylstannyl)thiophen-2-amine (0.40 g, 0.74 mmol), and Pd(PPh₃)₄ (0.04 g, 0.04 mmol) are dissolved in 150 mL of toluene and then, stirred at 110° C for 6 hours. When a reaction is complete, the toluene is evaporated so that the mixture is concentrated and the resultant is separated with a dichloromethane:hexane (1:1 v/v) column and then, precipitated in 100 mL of methanol to obtain Compound 7 (0.05 g). A yield is 20%.

NMR (300 MHz, CDCl3) : 7.67 (d, 2H), 7.40 (d, 2H), 7.36 (d, 8H), 7.30 (t, 8H), 7.22 (t, 4H), 7.16 (d, 2H), 6.58 (d, 2H).

### Comparative Synthesis Example 1

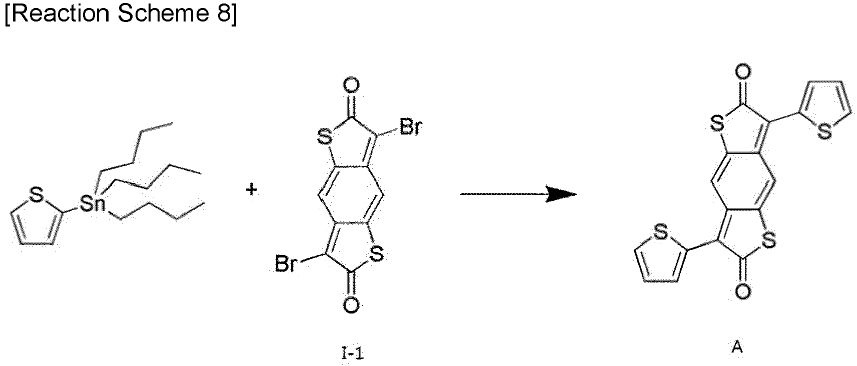

3,7-dibromobenzo[1,2-b:4,5-b']dithiophene-2,6-dione (Compound I-1) (0.30 g, 0.79 mmol), 2-(tributylstannyl)thiophene (0.62 g, 1.67 mmol), and Pd(PPh₃)₄ (0.09 g, 0.08 mmol) are dissolved in 50 mL of toluene and then, stirred at 110° C for 18 hours. When a reaction is complete, the toluene is evaporated so that the mixture is concentrated and the resultant is precipitated in 100 mL of methanol to obtain Compound A (0.2 g). A yield is 66%.

Confirmation of molecular weight: LC-MS: 384.97 m/z.

### Comparative Synthesis Example 2

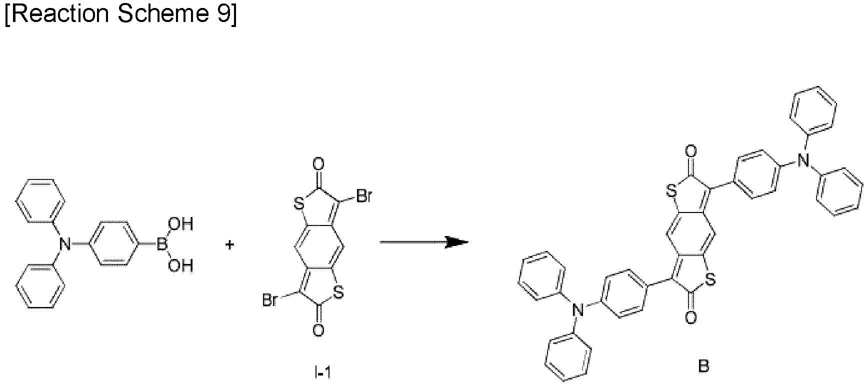

3,7-dibromobenzo[1,2-b:4,5-b']dithiophene-2,6-dione (Compound I-1) (0.90 g, 2.38 mmol), 4-(diphenylamino)phenylboronic acid (1.45 g, 5.01 mmol) and Pd(PPh₃)₄ (0.14 g, 0.12 mmol) are dissolved in 100 mL of toluene, 10 mL of a 1 M K₂CO₃ solution is added thereto, and then, the mixture is stirred at 100° C for 18 hours. When a reaction is complete, the resultant is extracted with dichloromethane and separated with dichloromethane:hexane (1:1 v/v) column, and precipitate in 100 mL of methanol to obtain Compound B (0.7 g). A yield is 42%.

Confirmation of molecular weight: LC-MS: 707.11 m/z.

### Evaluation I

The compounds of Synthesis Examples and Comparative Synthesis Examples are respectively dissolved at a concentration of 1×10⁻⁵ M in dichloromethane to prepare a solution, and light absorption characteristics of the compounds are evaluated.

The light absorption characteristics are evaluated by measuring a maximum absorption wavelength (λₘₐₓ) with a UV-Vis-NIR spectrometer (UV-3600 Plus, Shimadzu Corp.).

The results are shown in Table 1.

**Table 1**

| | λₘₐₓ (nm) |
|---|---|
| Synthesis Example 1 | 867 |
| Synthesis Example 2 | 802 |
| Synthesis Example 3 | 817 |
| Synthesis Example 4 | 834 |
| Synthesis Example 5 | 862 |
| Synthesis Example 6 | 844 |
| Synthesis Example 7 | 912 |
| Comparative Synthesis Example 1 | 572 |
| Comparative Synthesis Example 2 | 690 |

Referring to Table 1, the compounds according to the Synthesis Examples exhibit light absorption characteristics in long wavelength spectrums of greater than or equal to about 700 nm, for example greater than or equal to about 800 nm, compared with the compounds according to Comparative Synthesis Examples.

### Evaluation II

The compounds obtained in the Synthesis Examples are respectively dissolved at a concentration of 1×10⁻⁵ M in dichloromethane to prepare solutions and the light absorption characteristics of the compounds are evaluated.

Light absorption characteristics are obtained by using a Shimadzu UV-3600 Plus UV-Vis-NIR spectrometer to obtain absorption spectra and to determine the full widths at half maximum (FWHM).

The results are shown in Table 2.

**Table 2**

| | FWHM (nm) |
|---|---|
| Synthesis Example 1 | 168 |
| Synthesis Example 2 | 174 |
| Synthesis Example 3 | 167 |
| Synthesis Example 4 | 164 |
| Synthesis Example 5 | 123 |
| Synthesis Example 6 | 203 |
| Synthesis Example 7 | 187 |

Referring to Table 2, the compounds according to the Synthesis Examples exhibit good wavelength selectivity in the near infra-red wavelength spectrum.

### Evaluation III

Deposition characteristics of the compound of Synthesis Example are evaluated.

The deposition characteristics are evaluated by sublimating the compound under high vacuum of less than or equal to 10 Pa and measuring a weight loss depending on a temperature increase in a thermogravimetric analysis method.

The results are shown in Table 3.

**Table 3**

| | Tₛ(° C) (-10 wt%) | Tₛ (° C) (-50 wt%) |
|---|---|---|
| Synthesis Example 1 | 316 | 345 |
| Synthesis Example 2 | 325 | 366 |
| Synthesis Example 3 | 298 | - |
| Synthesis Example 4 | 261 | - |
| Synthesis Example 5 | 244 | - |
| Synthesis Example 6 | 348 | - |

| | | |
|---|---|---|
| * Tₛ (° C) (-10 wt%): a temperature at which a sample exhibits a 10 wt% weight loss * Tₛ(° C) (-50 wt%): a temperature at which a sample exhibits a 50 wt% weight loss Referring to Table 3, the compounds of Synthesis Examples have sufficient heat resistance. | | |

### Example

An about 150 nm-thick anode may be formed by sputtering ITO on a glass substrate. Subsequently, a 150 nm-thick photoelectric conversion layer may be formed by codepositing the compound of Synthesis Example 1 and C60 in a volume ratio of 1:1 on an anode. On the photoelectric conversion layer, an auxiliary layer may be formed by depositing C60. Then, a 7 nm-thick cathode may be formed on the auxiliary layer by sputtering ITO. Then, a 50 nm-thick anti-reflection layer may be formed on the cathode by depositing aluminum oxide (Al₂O₃) and it is sealed with a glass plate to manufacture a photoelectric diode.

### Evaluation IV

Photoelectric conversion efficiency of the photoelectric diode according to Example is evaluated.

The photoelectric conversion efficiency may be measured by using an IPCE measurement system (TNE Tech Co., Ltd., Korea). First, the IPCE measurement system is calibrated by using an Si photodiode (Hamamatsu Photonics K.K., Japan) and equipped with an organic photoelectric diode to measure external quantum efficiency in a wavelength spectrum of about 550 nm to about 1200 nm.

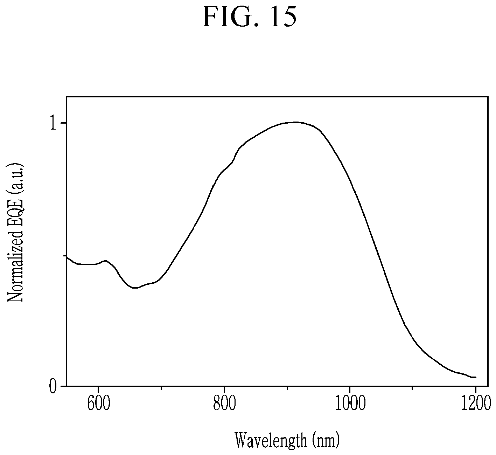

FIG. 15 is a graph showing photoelectric conversion efficiency of the photoelectric diode according to Example.

Referring to FIG. 15, the photoelectric diode according to Example exhibits good photoelectric conversion efficiency in a near infra-red wavelength spectrum of greater than or equal to about 800 nm.

While this disclosure has been described in connection with what is presently considered to be practical example embodiments, it is to be understood that the inventive concepts are not limited to the disclosed example embodiments. On the contrary, it is intended to cover various modifications and equivalent arrangements included within the scope of the appended claims.

## Claims

1. A compound represented by Chemical Formula 1: 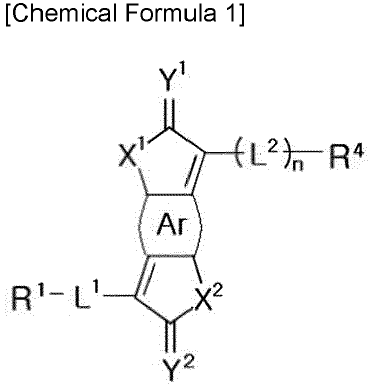 wherein,
X¹ and X² are each independently O, S, Se, or Te,
Y¹ and Y² are each independently O, S, Se, Te, or C(CN)₂,
Ar is a substituted or unsubstituted C6 to C30 aromatic ring, a substituted or unsubstituted C3 to C30 heteroaromatic ring, or a combination thereof,
L¹ and L² independently comprise at least one of the substituted or unsubstituted groups listed in Group 1: 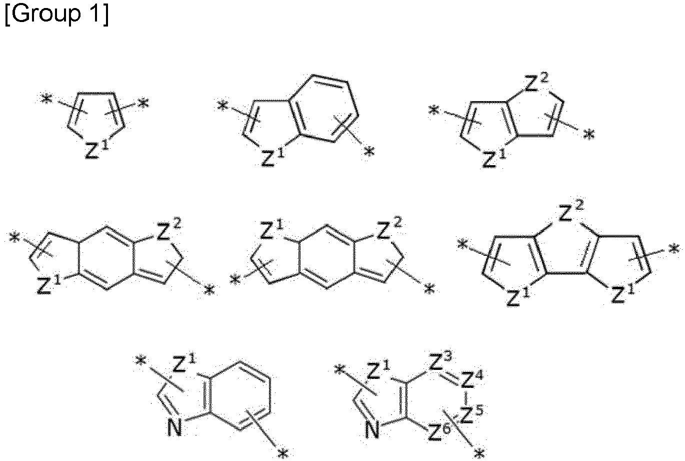
wherein,
Z¹ and Z² are each independently O, S, Se, Te, NR^{a}, or SiR^{b}R^{c},
Z³ to Z⁶ are each independently N or CR^{d},
at least one of Z³ to Z⁶ is N,
wherein R^{a} to R^{d} are each independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof, wherein R^{b} and R^{c} are each independently present or linked with each other to form a ring, and
* is a linking point.
R¹ is -NR²R³, wherein R² and R³ are each independently hydrogen, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted silyl group, a halogen, or a combination thereof, wherein R² and R³ are each independently present or linked with each other to form a ring,
R⁴ is represented by -NR⁵R⁶,
R⁵ and R⁶ are each independently hydrogen, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted silyl group, a halogen, or a combination thereof, wherein R⁵ and R⁶ are each independently present or linked with each other to form a ring, and
n is 0 or 1.

2. The compound of claim 1, wherein Ar is a substituted or unsubstituted benzene, a substituted or unsubstituted naphthalene, a substituted or unsubstituted anthracene, a substituted or unsubstituted phenanthrene, a substituted or unsubstituted tetracene, a substituted or unsubstituted pyrene, a substituted or unsubstituted thiophene, a substituted or unsubstituted furan, a substituted or unsubstituted selenophene, a substituted or unsubstituted tellurophene, or a combination of two or more thereof fused together.

3. The compound of claims 1 or 2, wherein R¹ is represented by one of Chemical Formulae A-1 to A-3: 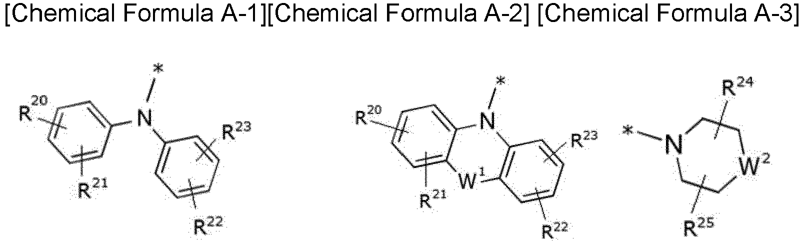
W¹ is a single bond, O, S, Se, NR^{e}, CR^{f}R^{g}, or SiR^{h}Rⁱ,
W² is O, S, Se, NR^{e}, CR^{f}R^{g}, or SiR^{h}Rⁱ,
R²⁰ to R²⁵ and R^{e} to Rⁱ are each independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof,
R²⁰ and R²¹ are each independently present or linked with each other to form a ring,
R²² and R²³ are each independently present or linked with each other to form a ring,
R²⁴ and R²⁵ are each independently present or linked with each other to form a ring,
R^{f} and R^{g} are each independently present or linked with each other to form a ring,
R^{h} and Rⁱ are each independently present or linked with each other to form a ring,
in Chemical Formula A-1, Chemical Formula A-2, or Chemical Formula A-3, at least one hydrogen in a ring structure is optionally replaced by a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted silyl group, a halogen, or a combination thereof, and
* is a linking point.

4. The compound of any of claims 1-3, wherein R⁴ is represented by one of Chemical Formulae B-1 to B-3: 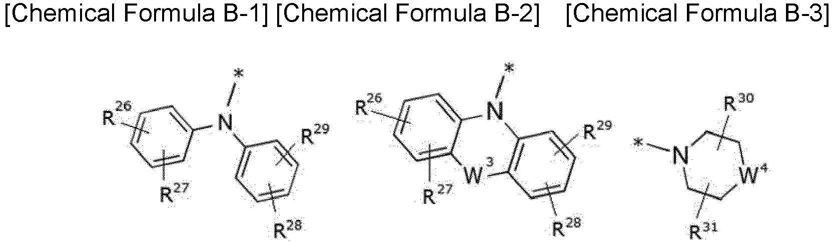 wherein,
W³ is a single bond, O, S, Se, NR^{j}, CR^{k}R^{l}, or SiR^{m}Rⁿ,
W⁴ is O, S, Se, NR^{j}, CR^{k}R^{l}, or SiR^{m}Rⁿ,
R²⁶ to R³¹ and R^{j} to Rⁿ are each independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof,
R²⁶ and R²⁷ are each independently present or linked with each other to form a ring,
R²⁸ and R²⁹ are each independently present or linked with each other to form a ring,
R³⁰ and R³¹ are each independently present or linked with each other to form a ring,
R^{k} and R^{l} are each independently present or linked with each other to form a ring,
R^{m} and Rⁿ are each independently present or linked with each other to form a ring,
in Chemical Formula B-1, Chemical Formula B-2, or Chemical Formula B-3, at least one hydrogen in the ring structure is optionally replaced by a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted silyl group, a halogen, or a combination thereof, and
* is a linking point.

5. The compound of any of claims 1-4, wherein the compound is represented by one of Chemical Formulae 1-J to 1-Z2: 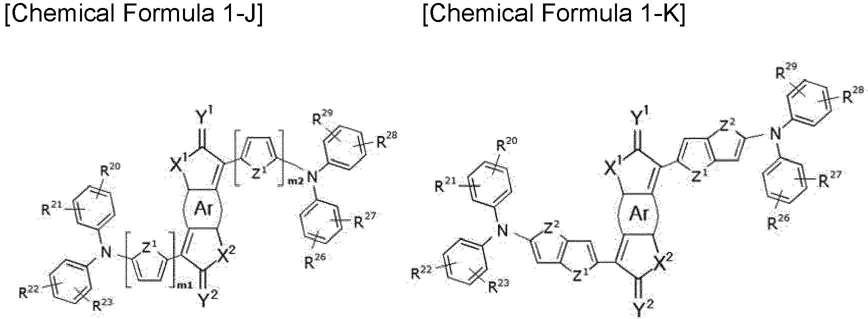 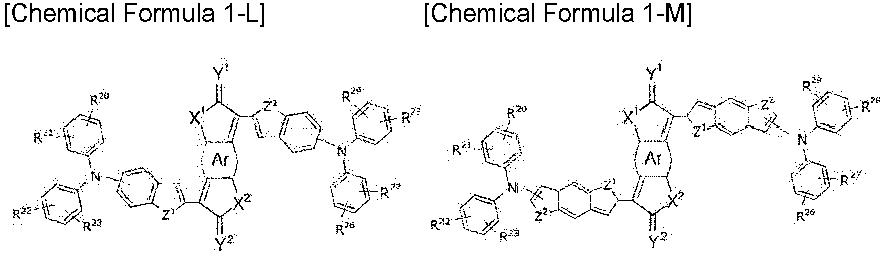 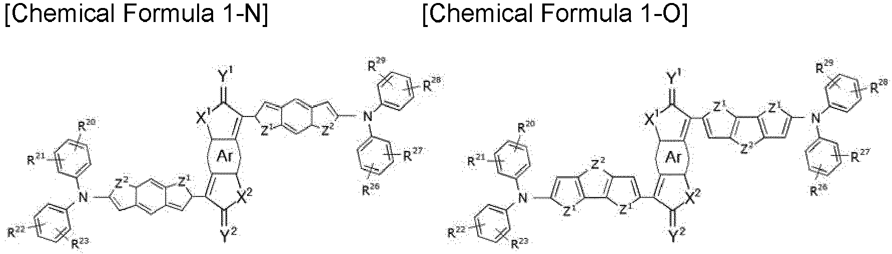 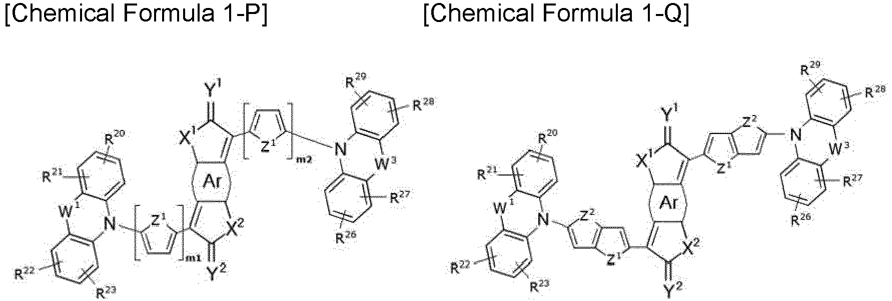 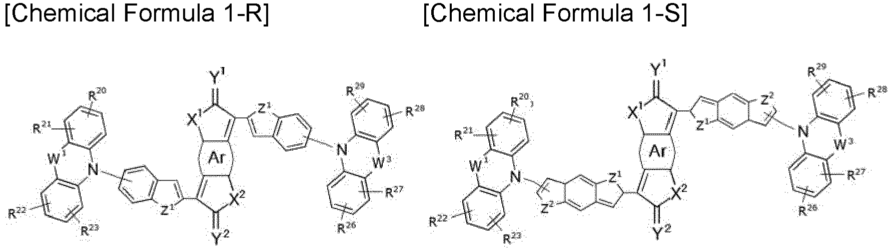 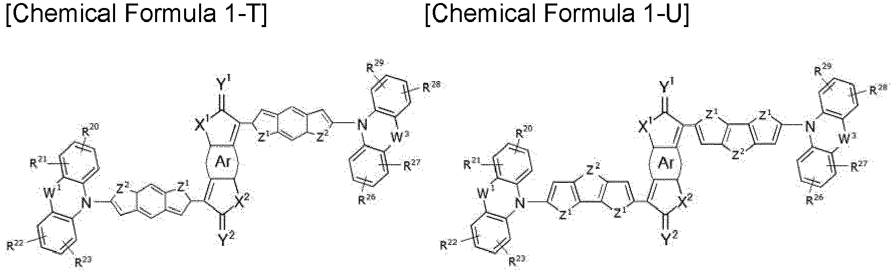 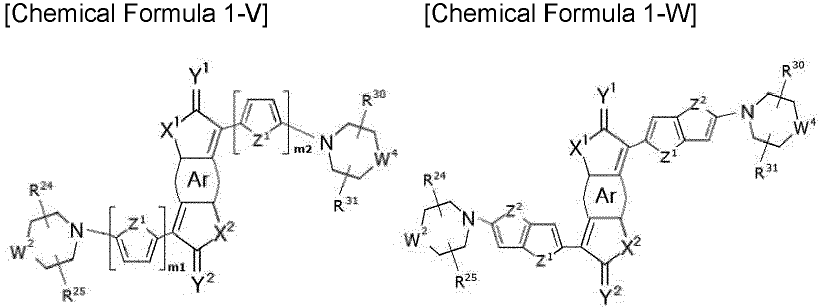 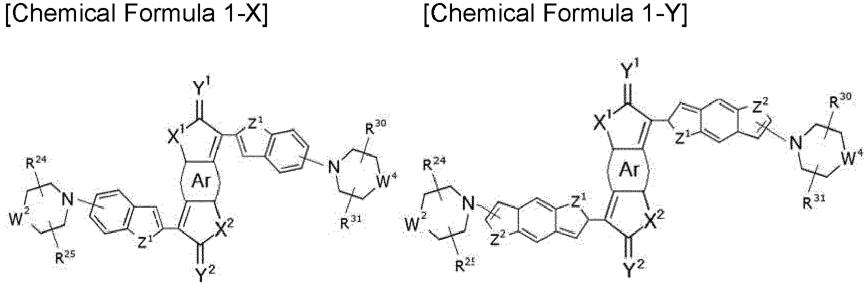 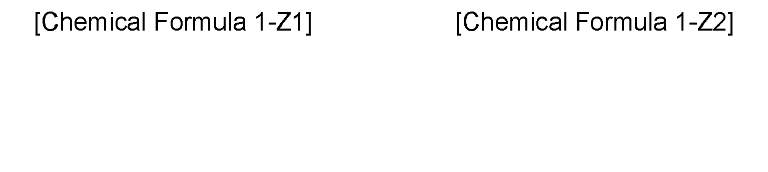 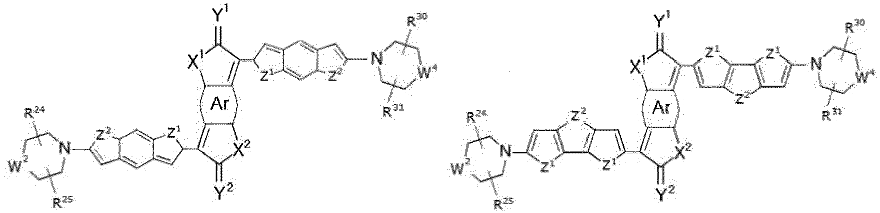 wherein,
X¹ and X² are each independently O, S, Se, or Te,
Y¹ and Y² are each independently O, S, Se, Te, or C(CN)₂,
Ar is a substituted or unsubstituted C6 to C30 aromatic ring, a substituted or unsubstituted C3 to C30 heteroaromatic ring, or a combination thereof,
Z¹ and Z² are each independently O, S, Se, Te, NR^{a}, or SiR^{b}R^{c},
W¹ is a single bond, O, S, Se, NR^{e}, CR^{f}R^{g}, or SiR^{h}Rⁱ,
W² is O, S, Se, NR^{e}, CR^{f}R^{g}, or SiR^{h}Rⁱ,
W³ is a single bond, O, S, Se, NR^{j}, CR^{k}R^{l}, or SiR^{m}Rⁿ,
W⁴ is O, S, Se, NR^{j}, CR^{k}R^{l}, or SiR^{m}Rⁿ,
R²⁰ to R³¹ and R^{a} to Rⁿ are each independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof,
R^{b} and R^{c} are each independently present or linked with each other to form a ring,
R^{f} and R^{g} are each independently present or linked with each other to form a ring,
R^{h} and Rⁱ are each independently present or linked with each other to form a ring,
R^{k} and R^{l} are each independently present or linked with each other to form a ring,
R^{m} and Rⁿ are each independently present or linked with each other to form a ring, and
m1 and m2 are each independently 1 to 5.

6. The compound of any of claims 1-5, wherein a peak absorption wavelength of the compound is within a wavelength spectrum of 700 nm to 3000 nm.

7. A film comprising the compound of any of claims 1-6.

8. A photoelectric diode comprising:
a first electrode and a second electrode facing each other, and
an organic layer between the first electrode and the second electrode
wherein the organic layer comprises a compound represented by Chemical Formula 1: 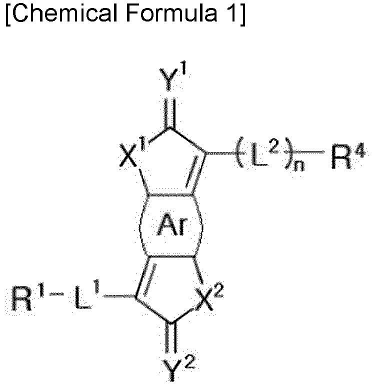
wherein,
X¹ and X² are each independently O, S, Se, or Te,
Y¹ and Y² are each independently O, S, Se, Te, or C(CN)₂,
Ar is a substituted or unsubstituted C6 to C30 aromatic ring, a substituted or unsubstituted C3 to C30 heteroaromatic ring, or a combination thereof,
L¹ and L² are each independently a substituted or unsubstituted C3 to C20 heteroarylene group,
R¹ is -NR²R³, wherein R² and R³ are each independently hydrogen, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted silyl group, a halogen, or a combination thereof, wherein R² and R³ are each independently present or linked with each other to form a ring,
R⁴ is represented by -NR⁵R⁶, wherein
R⁵ and R⁶ are each independently hydrogen, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted silyl group, a halogen, or a combination thereof, wherein R⁵ and R⁶ are each independently present or linked with each other to form a ring, and
n is 0 or 1.

9. The photoelectric diode of claim 8, wherein Ar is a substituted or unsubstituted benzene, a substituted or unsubstituted naphthalene, a substituted or unsubstituted anthracene, a substituted or unsubstituted phenanthrene, a substituted or unsubstituted tetracene, a substituted or unsubstituted pyrene, a substituted or unsubstituted thiophene, a substituted or unsubstituted furan, a substituted or unsubstituted selenophene, a substituted or unsubstituted tellurophene, or a combination of two or more thereof fused together.

10. The photoelectric diode of claims 8 or 9, wherein L¹ and L² independently comprise at least one of substituted or unsubstituted groups listed in Group 1: 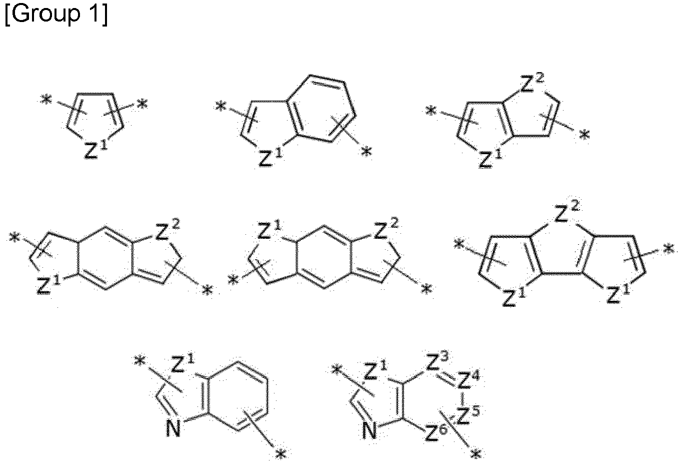 wherein,
Z¹ and Z² are each independently O, S, Se, Te, NR^{a}, or SiR^{b}R^{c},
Z³ to Z⁶ are each independently N or CR^{d},
at least one of Z³ to Z⁶ is N,
wherein R^{a} to R^{d} are each independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof, wherein R^{b} and R^{c} are each independently present or linked with each other to form a ring, and
* is a linking point.

11. The photoelectric dioide of any of claims 8-10, wherein R¹ is represented by one of Chemical Formulae A-1 to A-3: 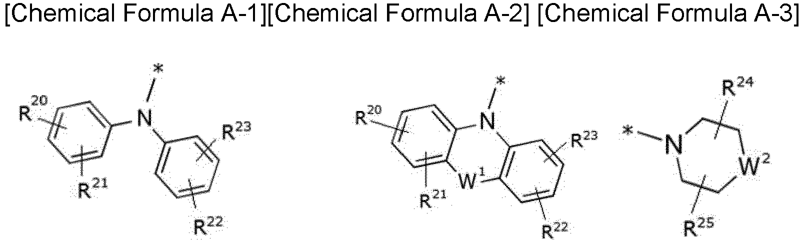
W¹ is a single bond, O, S, Se, NR^{e}, CR^{f}R^{g}, or SiR^{h}Rⁱ,
W² is O, S, Se, NR^{e}, CR^{f}R^{g}, or SiR^{h}Rⁱ,
R²⁰ to R²⁵ and R^{e} to Rⁱ are each independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof,
R²⁰ and R²¹ are each independently present or linked with each other to form a ring,
R²² and R²³ are each independently present or linked with each other to form a ring,
R²⁴ and R²⁵ are each independently present or linked with each other to form a ring,
R^{f} and R^{g} are each independently present or linked with each other to form a ring,
R^{h} and Rⁱ are each independently present or linked with each other to form a ring,
in Chemical Formula A-1, Chemical Formula A-2, or Chemical Formula A-3, at least one hydrogen in a ring structure is optionally replaced by a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted silyl group, a halogen, or a combination thereof,
* is a linking point, and
wherein R⁴ is represented by one of Chemical Formulae B-1 to B-3: 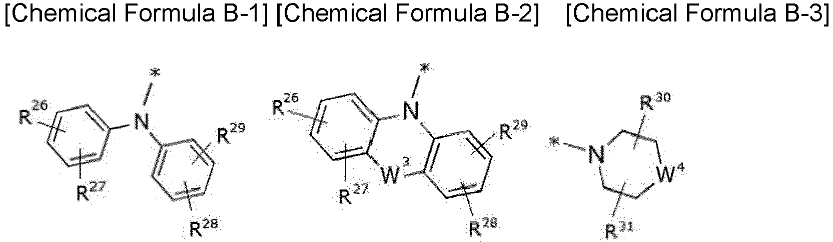
wherein,
W³ is a single bond, O, S, Se, NR^{j}, CR^{k}R^{l}, or SiR^{m}Rⁿ,
W⁴ is O, S, Se, NR^{j}, CR^{k}R^{l}, or SiR^{m}Rⁿ,
R²⁶ to R³¹ and R^{j} to Rⁿ are each independently hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted amine group, a substituted or unsubstituted silyl group, a halogen, or a combination thereof,
R²⁶ and R²⁷ are each independently present or linked with each other to form a ring,
R²⁸ and R²⁹ are each independently present or linked with each other to form a ring,
R³⁰ and R³¹ are each independently present or linked with each other to form a ring,
R^{k} and R^{l} are each independently present or linked with each other to form a ring,
R^{m} and Rⁿ are each independently present or linked with each other to form a ring,
in Chemical Formula B-1, Chemical Formula B-2, or Chemical Formula B-3, at least one hydrogen in the ring structure is optionally replaced by a substituted or unsubstituted C1 to C30 alkyl group, a substituted or unsubstituted C2 to C30 alkenyl group, a substituted or unsubstituted C2 to C30 alkynyl group, a substituted or unsubstituted C6 to C30 aryl group, a substituted or unsubstituted C3 to C30 heteroaryl group, a substituted or unsubstituted silyl group, a halogen, or a combination thereof, and
* is a linking point.

12. The photoelectric diode of any of claims 8-11, wherein the organic layer further comprises fullerene or a fullerene derivative.

13. The photoelectric diode of any of claims 8-12, wherein a peak absorption wavelength of the compound is within a wavelength spectrum of 700 nm to 3000 nm.

14. An organic sensor comprising the photoelectric diode of any of claims 8-13.

15. An electronic device comprising the organic sensor of claim 14 or the photoelectric diode of any of claims 8-13.

## Patentansprüche

1. Verbindung dargestellt durch die chemische Formel 1: 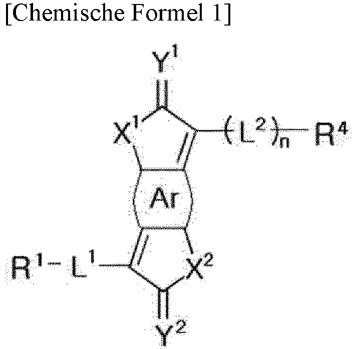 wobei
X¹ und X² jeweils unabhängig O, S, Se oder Te sind,
Y¹ und Y² jeweils unabhängig O, S, Se, Te oder C(CN)₂ sind,
Ar ein substituierter oder unsubstituierter aromatischer C6- bis C30-Ring, ein substituierter oder unsubstituierter heteroaromatischer C3- bis C30-Ring oder eine Kombination davon ist,
L¹ und L² unabhängig zumindest eine der substituierten oder unsubstituierten Gruppen umfassen, die in Gruppe 1 aufgelistet sind: 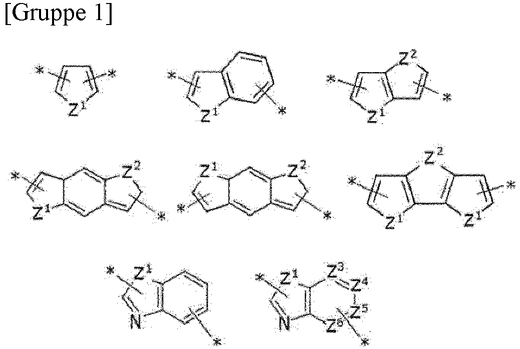
wobei
Z¹ und Z² jeweils unabhängig O, S, Se, Te, NR^{a} oder SiR^{b}R^{c} sind,
Z³ bis Z⁶ jeweils unabhängig N oder CR^{d} sind,
zumindest eines von Z³ bis Z⁶ N ist,
wobei R^{a}bis R^{d} jeweils unabhängig Wasserstoff, eine substituierte oder unsubstituierte C1-bis C30-Alkylgruppe, eine substituierte oder unsubstituierte C2- bis C30-Alkenylgruppe, eine substituierte oder unsubstituierte C2- bis C30-Alkynylgruppe, eine substituierte oder unsubstituierte C6- bis C30-Arylgruppe, eine substituierte oder unsubstituierte C3- bis C30-Heteroarylgruppe, eine substituierte oder unsubstituierte Amingruppe, eine substituierte oder unsubstituierte Silylgruppe, ein Halogen oder eine Kombination davon sind, wobei R^{b} und R^{c} jeweils unabhängig vorhanden oder miteinander verbunden sind, um einen Ring zu bilden, und
* ein Verbindungspunkt ist,
R¹ -NR²R³ ist, wobei R² und R³ jeweils unabhängig Wasserstoff, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C3- bis C30-Heteroarylgruppe, eine substituierte oder unsubstituierte Silylgruppe, ein Halogen oder eine Kombination davon sind, wobei R² und R³ jeweils unabhängig vorhanden oder miteinander verbunden sind, um einen Ring zu bilden,
R⁴ durch -NR⁵R⁶ dargestellt ist,
R⁵ und R⁶ jeweils unabhängig Wasserstoff, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C3- bis C30-Heteroarylgruppe, eine substituierte oder unsubstituierte Silylgruppe, ein Halogen oder eine Kombination davon sind, wobei R⁵ und R⁶ jeweils unabhängig vorhanden oder miteinander verbunden sind, um einen Ring zu bilden, und
n 0 oder 1 ist.

2. Verbindung nach Anspruch 1, wobei Ar ein substituiertes oder unsubstituiertes Benzol, ein substituiertes oder unsubstituiertes Naphthalen, ein substituiertes oder unsubstituiertes Anthracen, ein substituiertes oder unsubstituiertes Phenanthren, ein substituiertes oder unsubstituiertes Tetracen, ein substituiertes oder unsubstituiertes Pyren, ein substituiertes oder unsubstituiertes Thiophen, ein substituiertes oder unsubstituiertes Furan, ein substituiertes oder unsubstituiertes Selenophen, ein substituiertes oder unsubstituiertes Tellurophen oder eine Kombination von zwei oder mehr davon ist, die miteinander verschmolzen sind.

3. Verbindung nach Anspruch 1 oder 2, wobei R¹ durch eine der chemischen Formeln A-1 bis A-3 dargestellt ist: 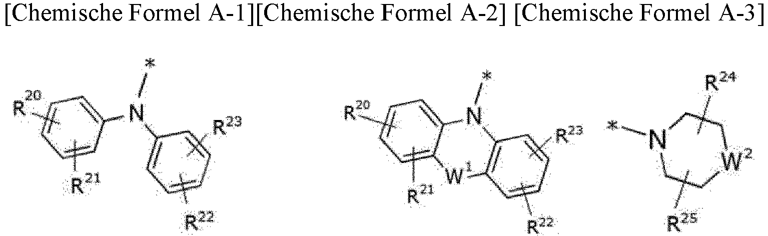
W¹ eine Einfachbindung, O, S, Se, NR^{e}, CR^{f}R^{g} oder SiR^{h}R' ist,
W² O, S, Se, NR^{e}, CR^{f}R^{g} oder SiR^{h}R' ist,
R²⁰ bis R²⁵ und R^{e} bis Rⁱ jeweils unabhängig Wasserstoff, eine substituierte oder unsubstituierte C1- bis C30-Alkylgruppe, eine substituierte oder unsubstituierte C2- bis C30-Alkenylgruppe, eine substituierte oder unsubstituierte C2- bis C30-Alkynylgruppe, eine substituierte oder unsubstituierte C6- bis C30-Arylgruppe, eine substituierte oder unsubstituierte C3- bis C30-Heteroarylgruppe, eine substituierte oder unsubstituierte Amingruppe, eine substituierte oder unsubstituierte Silylgruppe, ein Halogen oder eine Kombination davon sind,
R²⁰ und R²¹ jeweils unabhängig vorhanden oder miteinander verbunden sind, um einen Ring zu bilden,
R²² und R²³ jeweils unabhängig vorhanden oder miteinander verbunden sind, um einen Ring zu bilden,
R²⁴ und R²⁵ jeweils unabhängig vorhanden oder miteinander verbunden sind, um einen Ring zu bilden,
R^{f} und R^{g} jeweils unabhängig vorhanden oder miteinander verbunden sind, um einen Ring zu bilden,
R^{h} und Rⁱ jeweils unabhängig vorhanden oder miteinander verbunden sind, um einen Ring zu bilden,
in der chemischen Formel A-1, der chemischen Formel A-2 oder der chemischen Formel A-3 zumindest ein Wasserstoff in einer Ringstruktur optional durch eine substituierte oder unsubstituierte C1- bis C30-Alkylgruppe, eine substituierte oder unsubstituierte C2- bis C30-Alkenylgruppe, eine substituierte oder unsubstituierte C2- bis C30-Alkynylgruppe, eine substituierte oder unsubstituierte C6- bis C30-Arylgruppe, eine substituierte oder unsubstituierte C3- bis C30-Heteroarylgruppe, eine substituierte oder unsubstituierte Silylgruppe, ein Halogen oder eine Kombination davon ersetzt ist, und
* ein Verbindungspunkt ist.

4. Verbindung nach einem der Ansprüche 1-3, wobei R⁴ durch eine der chemischen Formeln B-1 bis B-3 dargestellt ist: 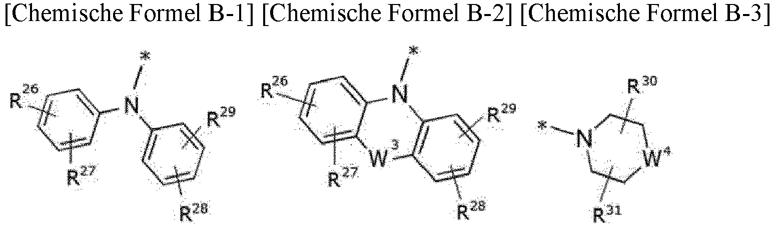 wobei
W³ eine Einfachbindung, O, S, Se, NR^{j}, CR^{k}R^{l} oder SiR^{m}Rⁿ ist,
W⁴ O, S, Se, NR^{j}, CR^{k}R^{l} oder SiR^{m}Rⁿ ist,
R²⁶ bis R³¹ und R^{j} bis Rⁿ jeweils unabhängig Wasserstoff, eine substituierte oder unsubstituierte C1- bis C30-Alkylgruppe, eine substituierte oder unsubstituierte C2- bis C30-Alkenylgruppe, eine substituierte oder unsubstituierte C2- bis C30-Alkynylgruppe, eine substituierte oder unsubstituierte C6- bis C30-Arylgruppe, eine substituierte oder unsubstituierte C3- bis C30-Heteroarylgruppe, eine substituierte oder unsubstituierte Amingruppe, eine substituierte oder unsubstituierte Silylgruppe, ein Halogen oder eine Kombination davon sind,
R²⁶ und R²⁷ jeweils unabhängig vorhanden oder miteinander verbunden sind, um einen Ring zu bilden,
R²⁸ und R²⁹ jeweils unabhängig vorhanden oder miteinander verbunden sind, um einen Ring zu bilden,
R³⁰ und R³¹ jeweils unabhängig vorhanden oder miteinander verbunden sind, um einen Ring zu bilden,
R^{k} und R^{l} jeweils unabhängig vorhanden oder miteinander verbunden sind, um einen Ring zu bilden,
R^{m} und Rⁿ jeweils unabhängig vorhanden oder miteinander verbunden sind, um einen Ring zu bilden,
in der chemischen Formel B-1, der chemischen Formel B-2 oder der chemischen Formel B-3 zumindest ein Wasserstoff in der Ringstruktur optional durch eine substituierte oder unsubstituierte C1- bis C30-Alkylgruppe, eine substituierte oder unsubstituierte C2- bis C30-Alkenylgruppe, eine substituierte oder unsubstituierte C2- bis C30-Alkynylgruppe, eine substituierte oder unsubstituierte C6- bis C30-Arylgruppe, eine substituierte oder unsubstituierte C3- bis C30-Heteroarylgruppe, eine substituierte oder unsubstituierte Silylgruppe, ein Halogen oder eine Kombination davon ersetzt ist, und
* ein Verbindungspunkt ist.

5. Verbindung nach einem der Ansprüche 1-4, wobei die Verbindung durch eine der chemischen Formeln 1-J bis 1-Z2 dargestellt ist: 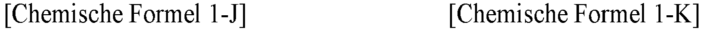 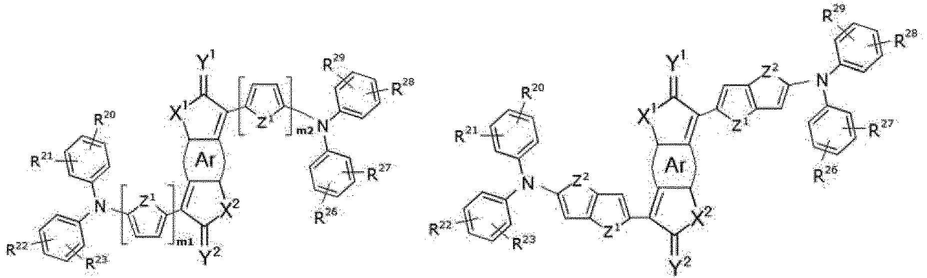 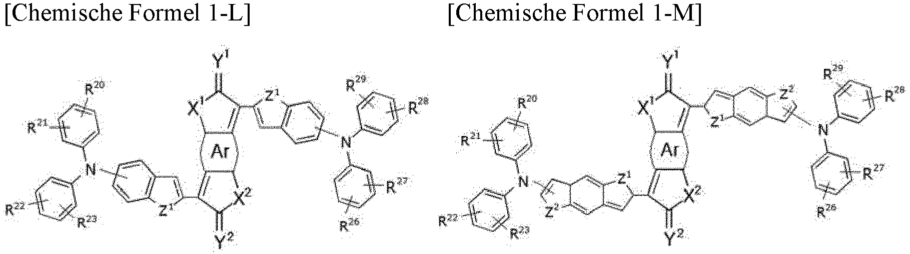 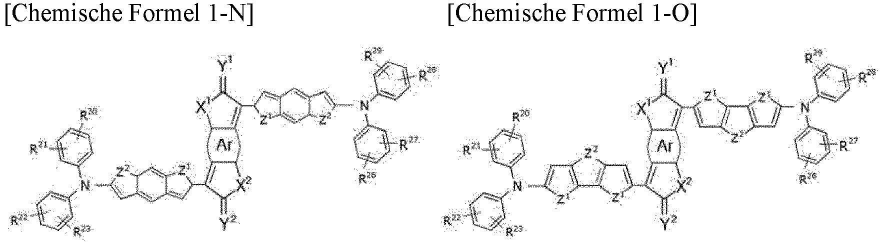 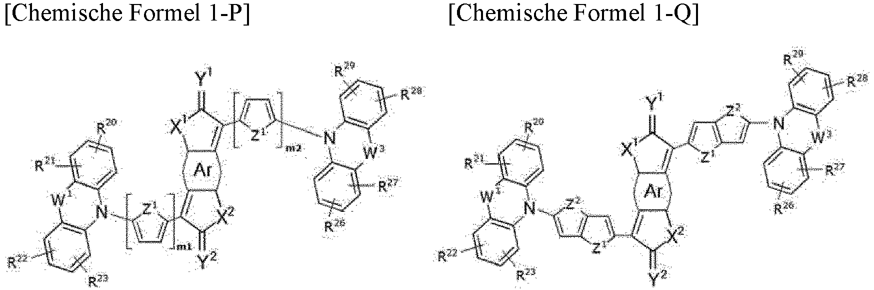 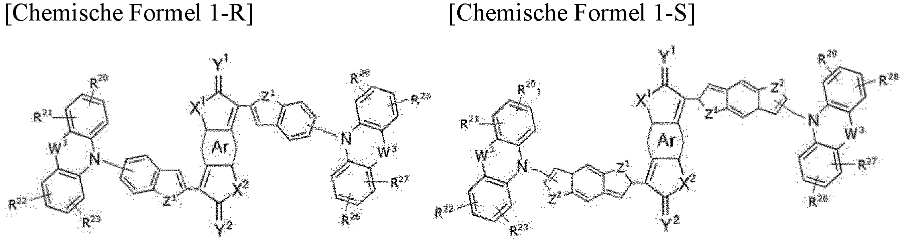 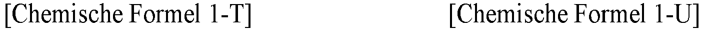 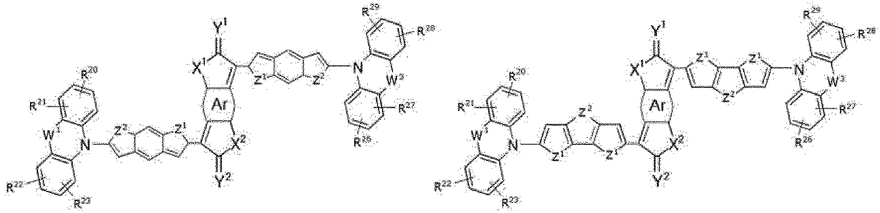 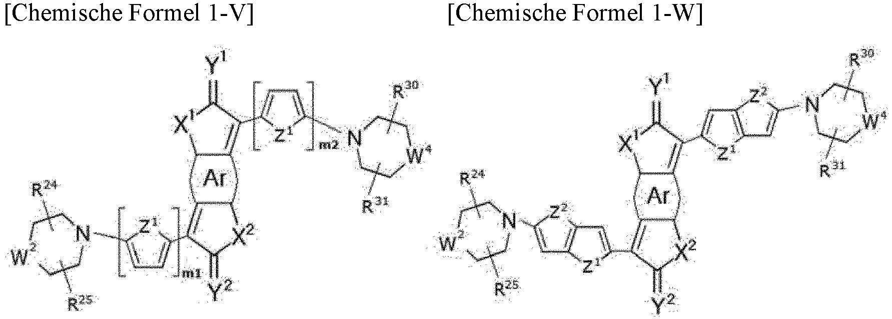 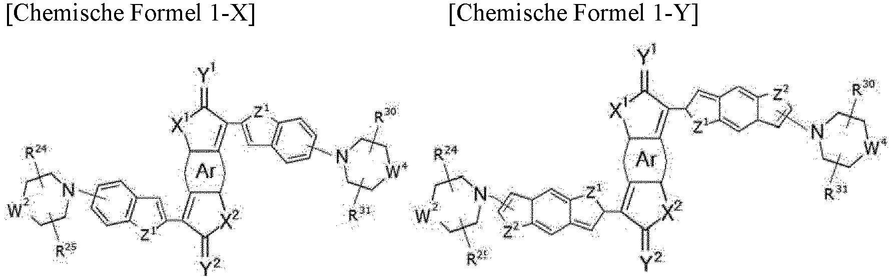 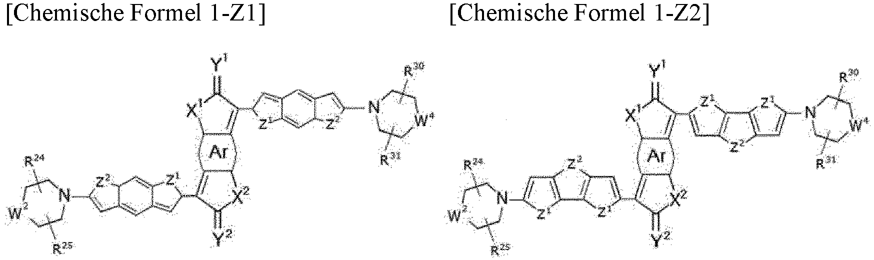 wobei
X¹ und X² jeweils unabhängig O, S, Se oder Te sind,
Y¹ und Y² jeweils unabhängig O, S, Se, Te oder C(CN)₂ sind,
Ar ein substituierter oder unsubstituierter aromatischer C6- bis C30-Ring, ein substituierter oder unsubstituierter heteroaromatischer C3- bis C30-Ring oder eine Kombination davon ist,
Z¹ und Z² jeweils unabhängig O, S, Se, Te, NR^{a} oder SiR^{b}R^{c} sind,
W¹ eine Einfachbindung, O, S, Se, NR^{e}, CR^{f}R^{g} oder SiR^{h}R' ist,
W² O, S, Se, NR^{e}, CR^{f}R^{g} oder SiR^{h}R' ist,
W³ eine Einfachbindung, O, S, Se, NR^{j}, CR^{k}R^{l} oder SiR^{m}Rⁿ ist,
W⁴ O, S, Se, NR^{j}, CR^{k}R^{l} oder SiR^{m}Rⁿ ist,
R²⁰ bis R³¹ und R^{a} bis Rⁿ jeweils unabhängig Wasserstoff, eine substituierte oder unsubstituierte C1- bis C30-Alkylgruppe, eine substituierte oder unsubstituierte C2- bis C30-Alkenylgruppe, eine substituierte oder unsubstituierte C2- bis C30-Alkynylgruppe, eine substituierte oder unsubstituierte C6- bis C30-Arylgruppe, eine substituierte oder unsubstituierte C3- bis C30-Heteroarylgruppe, eine substituierte oder unsubstituierte Amingruppe, eine substituierte oder unsubstituierte Silylgruppe, ein Halogen oder eine Kombination davon sind,
R^{b} und R^{c} jeweils unabhängig vorhanden oder miteinander verbunden sind, um einen Ring zu bilden,
R^{f} und R^{g} jeweils unabhängig vorhanden oder miteinander verbunden sind, um einen Ring zu bilden,
R^{h} und Rⁱ jeweils unabhängig vorhanden oder miteinander verbunden sind, um einen Ring zu bilden,
R^{k} und R^{l} jeweils unabhängig vorhanden oder miteinander verbunden sind, um einen Ring zu bilden,
R^{m} und Rⁿ jeweils unabhängig vorhanden oder miteinander verbunden sind, um einen Ring zu bilden, und
m1 und m2 jeweils unabhängig 1 bis 5 sind.

6. Verbindung nach einem der Ansprüche 1-5, wobei eine Spitzenabsorptionswellenlänge der Verbindung innerhalb eines Wellenlängenspektrums von 700 nm bis 3000 nm ist.

7. Folie, umfassend die Verbindung nach einem der Ansprüche 1-6.

8. Photoelektrische Diode, umfassend:
eine erste Elektrode und eine zweite Elektrode, die einander zugewandt sind, und
eine organische Schicht zwischen der ersten Elektrode und der zweiten Elektrode,
wobei die organische Schicht eine Verbindung dargestellt durch die chemische Formel 1 umfasst: 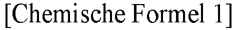 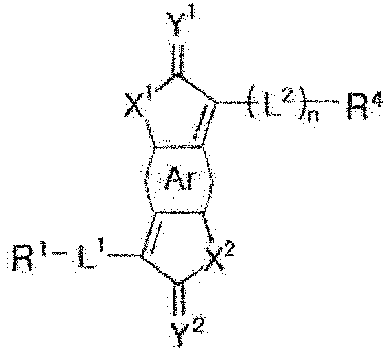
wobei
X¹ und X² jeweils unabhängig O, S, Se oder Te sind,
Y¹ und Y² jeweils unabhängig O, S, Se, Te oder C(CN)₂ sind,
Ar ein substituierter oder unsubstituierter aromatischer C6- bis C30-Ring, ein substituierter oder unsubstituierter heteroaromatischer C3- bis C30-Ring oder eine Kombination davon ist,
L¹ und L² jeweils unabhängig eine substituierte oder unsubstituierte C3- bis C20-Heteroarylengruppe sind,
R¹ -NR²R³ ist, wobei R² und R³ jeweils unabhängig Wasserstoff, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C3- bis C30-Heteroarylgruppe, eine substituierte oder unsubstituierte Silylgruppe, ein Halogen oder eine Kombination davon sind, wobei R² und R³ jeweils unabhängig vorhanden oder miteinander verbunden sind, um einen Ring zu bilden,
R⁴ durch -NR⁵R⁶ dargestellt ist, wobei
R⁵ und R⁶ jeweils unabhängig Wasserstoff, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C3- bis C30-Heteroarylgruppe, eine substituierte oder unsubstituierte Silylgruppe, ein Halogen oder eine Kombination davon sind, wobei R⁵ und R⁶ jeweils unabhängig vorhanden oder miteinander verbunden sind, um einen Ring zu bilden, und
n 0 oder 1 ist.

9. Photoelektrische Diode nach Anspruch 8, wobei Ar ein substituiertes oder unsubstituiertes Benzol, ein substituiertes oder unsubstituiertes Naphthalen, ein substituiertes oder unsubstituiertes Anthracen, ein substituiertes oder unsubstituiertes Phenanthren, ein substituiertes oder unsubstituiertes Tetracen, ein substituiertes oder unsubstituiertes Pyren, ein substituiertes oder unsubstituiertes Thiophen, ein substituiertes oder unsubstituiertes Furan, ein substituiertes oder unsubstituiertes Selenophen, ein substituiertes oder unsubstituiertes Tellurophen oder eine Kombination von zwei oder mehr davon ist, die miteinander verschmolzen sind.

10. Photoelektrische Diode nach Anspruch 8 oder 9, wobei L¹ und L² unabhängig zumindest eine der substituierten oder unsubstituierten Gruppen umfassen, die in Gruppe 1 aufgelistet sind: 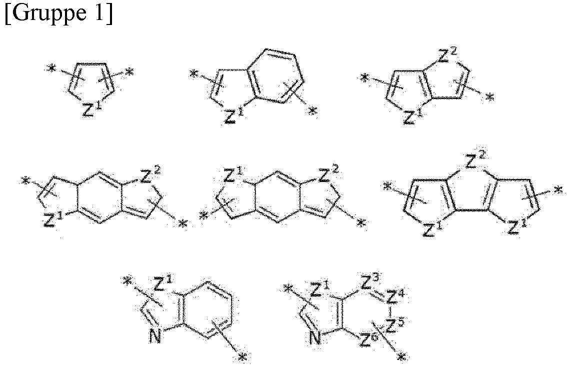 wobei
Z¹ und Z² jeweils unabhängig O, S, Se, Te, NR^{a} oder SiR^{b}R^{c} sind,
Z³ bis Z⁶ jeweils unabhängig N oder CR^{d} sind,
zumindest eines von Z³ bis Z⁶ N ist,
wobei R^{a} bis R^{d} jeweils unabhängig Wasserstoff, eine substituierte oder unsubstituierte C1-bis C30-Alkylgruppe, eine substituierte oder unsubstituierte C2- bis C30-Alkenylgruppe, eine substituierte oder unsubstituierte C2- bis C30-Alkynylgruppe, eine substituierte oder unsubstituierte C6- bis C30-Arylgruppe, eine substituierte oder unsubstituierte C3- bis C30-Heteroarylgruppe, eine substituierte oder unsubstituierte Amingruppe, eine substituierte oder unsubstituierte Silylgruppe, ein Halogen oder eine Kombination davon sind, wobei R^{b} und R^{c} jeweils unabhängig vorhanden oder miteinander verbunden sind, um einen Ring zu bilden, und
* ein Verbindungspunkt ist.

11. Photoelektrische Diode nach einem der Ansprüche 8-10, wobei R¹ durch eine der chemischen Formeln A-1 bis A-3 dargestellt ist: 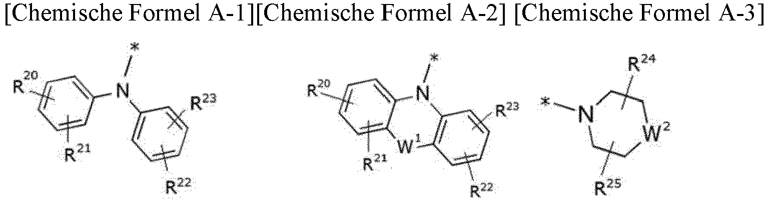
W¹ eine Einfachbindung, O, S, Se, NR^{e}, CR^{f}R^{g} oder SiR^{h}R' ist,
W² O, S, Se, NR^{e}, CR^{f}R^{g} oder SiR^{h}Rⁱ ist,
R²⁰ bis R²⁵ und R^{e} bis Rⁱ jeweils unabhängig Wasserstoff, eine substituierte oder unsubstituierte C1- bis C30-Alkylgruppe, eine substituierte oder unsubstituierte C2- bis C30-Alkenylgruppe, eine substituierte oder unsubstituierte C2- bis C30-Alkynylgruppe, eine substituierte oder unsubstituierte C6- bis C30-Arylgruppe, eine substituierte oder unsubstituierte C3- bis C30-Heteroarylgruppe, eine substituierte oder unsubstituierte Amingruppe, eine substituierte oder unsubstituierte Silylgruppe, ein Halogen oder eine Kombination davon sind,
R²⁰ und R²¹ jeweils unabhängig vorhanden oder miteinander verbunden sind, um einen Ring zu bilden,
R²² und R²³ jeweils unabhängig vorhanden oder miteinander verbunden sind, um einen Ring zu bilden,
R²⁴ und R²⁵ jeweils unabhängig vorhanden oder miteinander verbunden sind, um einen Ring zu bilden,
R^{f} und R^{g} jeweils unabhängig vorhanden oder miteinander verbunden sind, um einen Ring zu bilden,
R^{h} und Rⁱ jeweils unabhängig vorhanden oder miteinander verbunden sind, um einen Ring zu bilden,
in der chemischen Formel A-1, der chemischen Formel A-2 oder der chemischen Formel A-3 zumindest ein Wasserstoff in einer Ringstruktur optional durch eine substituierte oder unsubstituierte C1- bis C30-Alkylgruppe, eine substituierte oder unsubstituierte C2- bis C30-Alkenylgruppe, eine substituierte oder unsubstituierte C2- bis C30-Alkynylgruppe, eine substituierte oder unsubstituierte C6- bis C30-Arylgruppe, eine substituierte oder unsubstituierte C3- bis C30-Heteroarylgruppe, eine substituierte oder unsubstituierte Silylgruppe, ein Halogen oder eine Kombination davon ersetzt ist,
* ein Verbindungspunkt ist; und
wobei R⁴ durch eine der chemischen Formeln B-1 bis B-3 dargestellt ist: 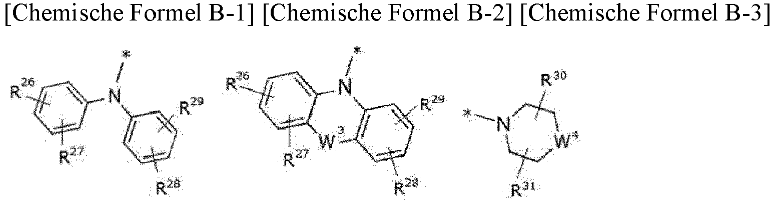
wobei
W³ eine Einfachbindung, O, S, Se, NR^{j}, CR^{k}R^{l} oder SiR^{m}Rⁿ ist,
W⁴ O, S, Se, NR^{j}, CR^{k}R^{l} oder SiR^{m}Rⁿ ist,
R²⁶ bis R³¹ und R^{j} bis Rⁿ jeweils unabhängig Wasserstoff, eine substituierte oder unsubstituierte C1- bis C30-Alkylgruppe, eine substituierte oder unsubstituierte C2- bis C30-Alkenylgruppe, eine substituierte oder unsubstituierte C2- bis C30-Alkynylgruppe, eine substituierte oder unsubstituierte C6- bis C30-Arylgruppe, eine substituierte oder unsubstituierte C3- bis C30-Heteroarylgruppe, eine substituierte oder unsubstituierte Amingruppe, eine substituierte oder unsubstituierte Silylgruppe, ein Halogen oder eine Kombination davon sind,
R²⁶ und R²⁷ jeweils unabhängig vorhanden oder miteinander verbunden sind, um einen Ring zu bilden,
R²⁸ und R²⁹ jeweils unabhängig vorhanden oder miteinander verbunden sind, um einen Ring zu bilden,
R³⁰ und R³¹ jeweils unabhängig vorhanden oder miteinander verbunden sind, um einen Ring zu bilden,
R^{k} und R^{l} jeweils unabhängig vorhanden oder miteinander verbunden sind, um einen Ring zu bilden,
R^{m} und Rⁿ jeweils unabhängig vorhanden oder miteinander verbunden sind, um einen Ring zu bilden,
in der chemischen Formel B-1, der chemischen Formel B-2 oder der chemischen Formel B-3 zumindest ein Wasserstoff in der Ringstruktur optional durch eine substituierte oder unsubstituierte C1- bis C30-Alkylgruppe, eine substituierte oder unsubstituierte C2- bis C30-Alkenylgruppe, eine substituierte oder unsubstituierte C2- bis C30-Alkynylgruppe, eine substituierte oder unsubstituierte C6- bis C30-Arylgruppe, eine substituierte oder unsubstituierte C3- bis C30-Heteroarylgruppe, eine substituierte oder unsubstituierte Silylgruppe, ein Halogen oder eine Kombination davon ersetzt ist, und
* ein Verbindungspunkt ist.

12. Photoelektrische Diode nach einem der Ansprüche 8-11, wobei die organische Schicht ferner Fulleren oder ein Fulleren-Derivat umfasst.

13. Photoelektrische Diode nach einem der Ansprüche 8-12, wobei eine Spitzenabsorptionswellenlänge der Verbindung innerhalb eines Wellenlängenspektrums von 700 nm bis 3000 nm ist.

14. Organischer Sensor, umfassend die photoelektrische Diode nach einem der Ansprüche 8-13.

15. Elektronische Vorrichtung, umfassend den organischen Sensor nach Anspruch 14 oder die photoelektrische Diode nach einem der Ansprüche 8-13.

## Revendications

1. Composé représenté par la formule chimique 1 : 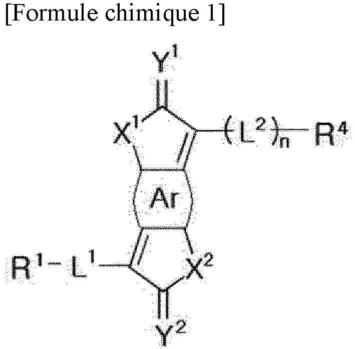 dans laquelle,
X¹ et X² représentent chacun indépendamment un atome O, S, Se ou Te,
Y¹ et Y² représentent chacun indépendamment un atome O, S, Se, Te, ou un groupe C(CN)₂,
Ar représente un noyau aromatique en C6 à C30 substitué ou non substitué, un noyau hétéroaromatique en C3 à C30 substitué ou non substitué, ou une combinaison de ceux-ci,
L¹ et L² comprennent indépendamment au moins l'un des groupes substitués ou non substitués énumérés dans le groupe 1 : 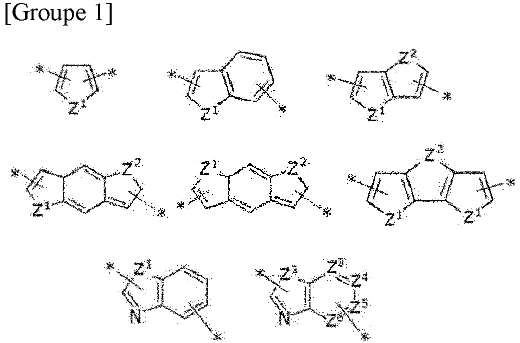
dans lesquels,
Z¹ et Z² représentent chacun indépendamment un atome O, S, Se, Te, un groupe NR^{a} ou SiR^{b}R^{c},
Z³ à Z⁶ représentent chacun indépendamment un atome N ou un groupe CR^{d},
au moins l'un des groupes Z³ à Z⁶ représente un atome N,
R^{a} à R^{d} représentant chacun indépendamment un atome d'hydrogène, un groupe alkyle en C1 à C30 substitué ou non substitué, un groupe alcényle en C2 à C30 substitué ou non substitué, un groupe alcynyle en C2 à C30 substitué ou non substitué, un groupe aryle en C6 à C30 substitué ou non substitué, un groupe hétéroaryle en C3 à C30 substitué ou non substitué, un groupe amine substitué ou non substitué, un groupe silyle substitué ou non substitué, un atome d'halogène, ou une combinaison de ceux-ci, R^{b} et R^{c} étant chacun indépendamment présents ou liés l'un à l'autre pour former un noyau, et
* représente un point de liaison,
R¹ représente un groupe -NR²R³, R² et R³ représentant chacun indépendamment un atome d'hydrogène, un groupe alkyle en C1 à C30 substitué ou non substitué, un groupe alcoxy en C1 à C30 substitué ou non substitué, un groupe aryle en C6 à C30 substitué ou non substitué, un groupe hétéroaryle en C3 à C30 substitué ou non substitué, un groupe silyle substitué ou non substitué, un atome d'halogène, ou une combinaison de ceux-ci, R² et R³ étant chacun indépendamment présents ou liés l'un à l'autre pour former un noyau,
R⁴ est représenté par un groupe -NR⁵R⁶,
R⁵ et R⁶ représentent chacun indépendamment un atome d'hydrogène, un groupe alkyle en C1 à C30 substitué ou non substitué, un groupe alcoxy en C1 à C30 substitué ou non substitué, un groupe aryle en C6 à C30 substitué ou non substitué, un groupe hétéroaryle en C3 à C30 substitué ou non substitué, un groupe silyle substitué ou non substitué, un atome d'halogène, ou une combinaison de ceux-ci, R⁵ et R⁶ étant chacun indépendamment présents ou liés l'un à l'autre pour former un noyau, et
n vaut 0 ou 1.

2. Composé selon la revendication 1, Ar représentant un noyau benzène substitué ou non substitué, un noyau naphtalène substitué ou non substitué, un noyau anthracène substitué ou non substitué, un noyau phénanthrène substitué ou non substitué, un noyau tétracène substitué ou non substitué, un noyau pyrène substitué ou non substitué, un noyau thiophène substitué ou non substitué, un noyau furane substitué ou non substitué, un noyau sélénophène substitué ou non substitué, un noyau tellurophène substitué ou non substitué, ou une combinaison d'au moins deux noyaux parmi ceux-ci fusionnés ensemble.

3. Composé selon la revendication 1 ou 2, R¹ étant représenté par l'une des formules chimiques A-1 à A-3 : 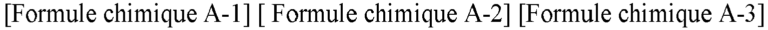 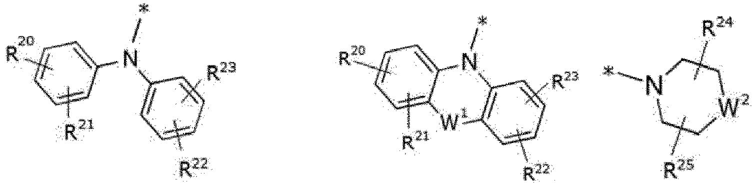
W¹ représentant une liaison simple, un atome O, S, Se, un groupe NR^{e}, CR^{f}R^{g} ou SiR^{h}Rⁱ,
W² représentant un atome O, S, Se, un groupe NR^{e}, CR^{f}R^{g} ou SiR^{h}Rⁱ,
R²⁰ à R²⁵ et R^{e} à Rⁱ représentant chacun indépendamment un atome d'hydrogène, un groupe alkyle en C1 à C30 substitué ou non substitué, un groupe alcényle en C2 à C30 substitué ou non substitué, un groupe alcynyle en C2 à C30 substitué ou non substitué, un groupe aryle en C6 à C30 substitué ou non substitué, un groupe hétéroaryle en C3 à C30 substitué ou non substitué, un groupe amine substitué ou non substitué, un groupe silyle substitué ou non substitué, un atome d'halogène, ou une combinaison de ceux-ci,
R²⁰ et R²¹ étant chacun indépendamment présents ou liés l'un à l'autre pour former un noyau,
R²² et R²³ étant chacun indépendamment présents ou liés l'un à l'autre pour former un noyau,
R²⁴ et R²⁵ étant chacun indépendamment présents ou liés l'un à l'autre pour former un noyau,
R^{f} et R^{g} étant chacun indépendamment présents ou liés l'un à l'autre pour former un noyau,
R^{h} et Rⁱ étant chacun indépendamment présents ou liés l'un à l'autre pour former un noyau,
dans la formule chimique A-1, dans la formule chimique A-2 ou dans la formule chimique A-3, au moins un atome d'hydrogène dans une structure de noyau étant éventuellement remplacé par un groupe alkyle en C1 à C30 substitué ou non substitué, un groupe alcényle en C2 à C30 substitué ou non substitué, un groupe alcynyle en C2 à C30 substitué ou non substitué, un groupe aryle en C6 à C30 substitué ou non substitué, un groupe hétéroaryle en C3 à C30 substitué ou non substitué, un groupe silyle substitué ou non substitué, un atome d'halogène, ou une combinaison de ceux-ci, et
* représentant un point de liaison.

4. Composé selon l'une quelconque des revendications 1 à 3, R⁴ étant représenté par l'une des formules chimique B-1 à B-3 : 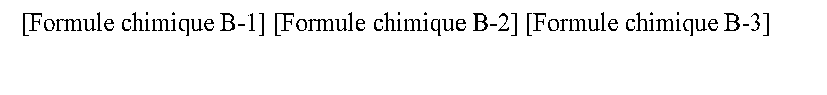 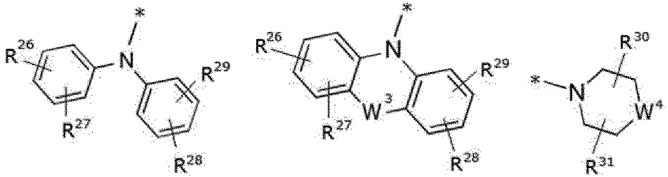 dans lesquelles,
W³ représente une liaison simple, un atome O, S, Se, un groupe NR^{j}, CR^{k}R^{l} ou SiR^{m}Rⁿ,
W⁴ représente un atome O, S, Se, un groupe NR^{j}, CR^{k}R^{l} ou SiR^{m}Rⁿ,
R²⁶ à R³¹ et R^{j} à Rⁿ représentent chacun indépendamment un atome d'hydrogène, un groupe alkyle en C1 à C30 substitué ou non substitué, un groupe alcényle en C2 à C30 substitué ou non substitué, un groupe alcynyle en C2 à C30 substitué ou non substitué, un groupe aryle en C6 à C30 substitué ou non substitué, un groupe hétéroaryle en C3 à C30 substitué ou non substitué, un groupe amine substitué ou non substitué, un groupe silyle substitué ou non substitué, un atome d'halogène, ou une combinaison de ceux-ci,
R²⁶ et R²⁷ sont chacun indépendamment présents ou liés l'un à l'autre pour former un noyau,
R²⁸ et R²⁹ sont chacun indépendamment présents ou liés l'un à l'autre pour former un noyau,
R³⁰ et R³¹ sont chacun indépendamment présents ou liés l'un à l'autre pour former un noyau,
R^{k} et R^{l} sont chacun indépendamment présents ou liés l'un à l'autre pour former un noyau,
R^{m} et Rⁿ sont chacun indépendamment présents ou liés l'un à l'autre pour former un noyau,
dans la formule chimique B-1, la formule chimique B-2 ou la formule chimique B-3, au moins un atome d'hydrogène dans la structure de noyau est éventuellement remplacé par un groupe alkyle en C1 à C30 substitué ou non substitué, un groupe alcényle en C2 à C30 substitué ou non substitué, un groupe alcynyle en C2 à C30 substitué ou non substitué, un groupe aryle en C6 à C30 substitué ou non substitué, un groupe hétéroaryle en C3 à C30 substitué ou non substitué, un groupe silyle substitué ou non substitué, un atome d'halogène, ou une combinaison de ceux-ci, et
* représente un point de liaison.

5. Composé selon l'une quelconque des revendications 1 à 4, ledit composé étant représenté par l'une des formules chimiques 1-J à 1-Z2 : 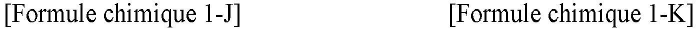 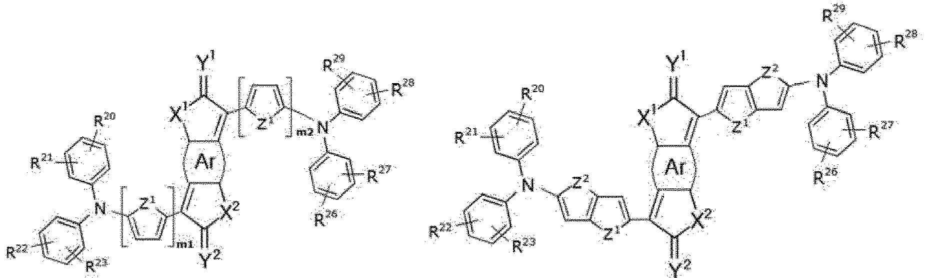 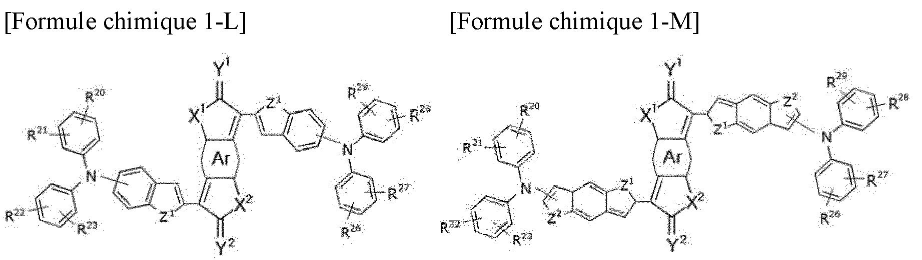 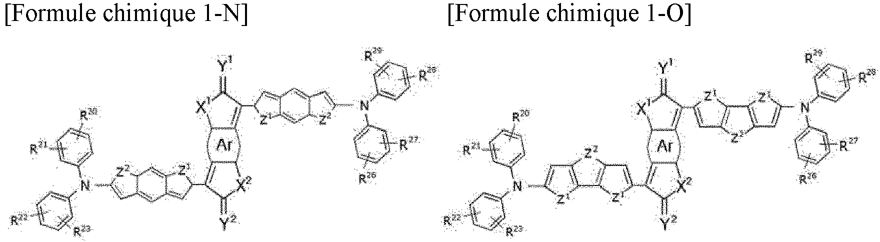 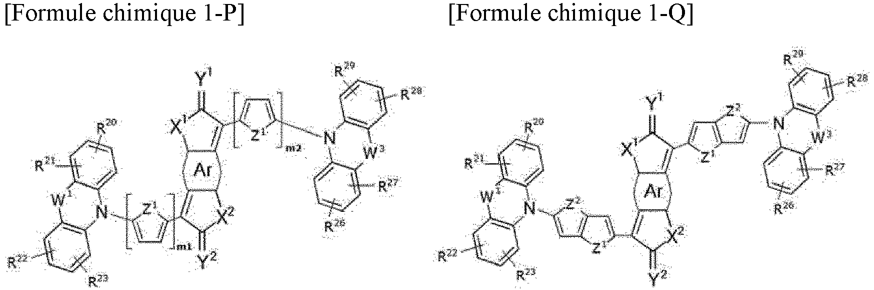 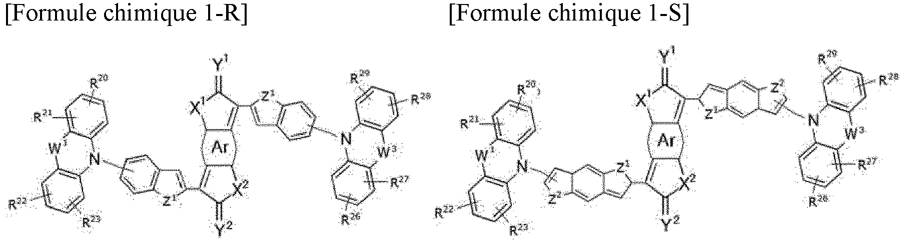 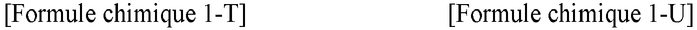 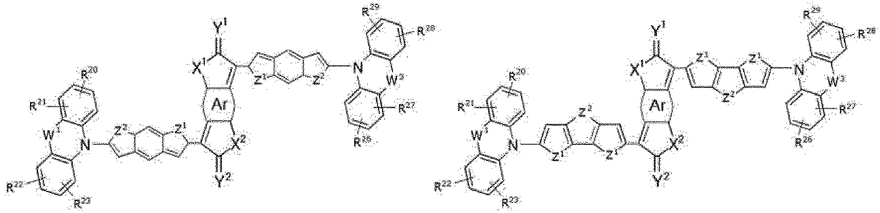 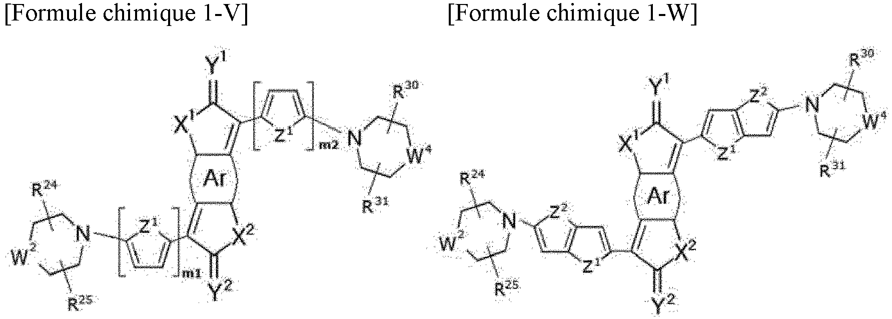 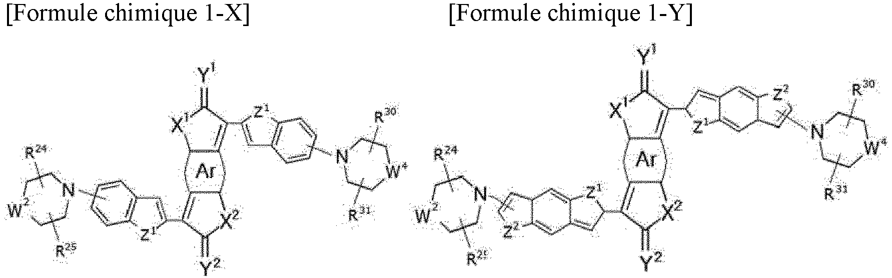 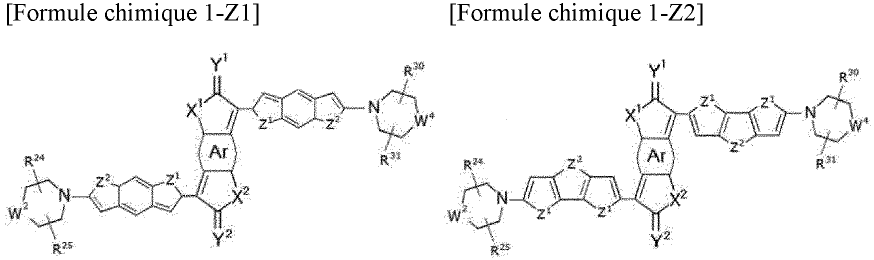 dans lesquelles,
X¹ et X² représentent chacun indépendamment un atome O, S, Se ou Te,
Y¹ et Y² représentent chacun indépendamment un atome O, S, Se, Te, ou un groupe C(CN)₂,
Ar représente un noyau aromatique en C6 à C30 substitué ou non substitué, un noyau hétéroaromatique en C3 à C30 substitué ou non substitué, ou une combinaison de ceux-ci,
Z¹ et Z² représentent chacun indépendamment un atome O, S, Se, Te, un groupe NR^{a} ou SiR^{b}R^{c},
W¹ représente une liaison simple, un atome O, S, Se, un groupe NR^{e}, CR^{f}R^{g} ou SiR^{h}Rⁱ,
W² représente un atome O, S, Se, un groupe NR^{e}, CR^{f}R^{g} ou SiR^{h}Rⁱ,
W³ représente une liaison simple, un atome O, S, Se, un groupe NR^{j}, CR^{k}R^{l} ou SiR^{m}Rⁿ,
W⁴ représente un atome O, S, Se, un groupe NR^{j}, CR^{k}R^{l} ou SiR^{m}Rⁿ,
R²⁰ à R³¹ et R^{a} à Rⁿ représentent chacun indépendamment un atome d'hydrogène, un groupe alkyle en C1 à C30 substitué ou non substitué, un groupe alcényle en C2 à C30 substitué ou non substitué, un groupe alcynyle en C2 à C30 substitué ou non substitué, un groupe aryle en C6 à C30 substitué ou non substitué, un groupe hétéroaryle en C3 à C30 substitué ou non substitué, un groupe amine substitué ou non substitué, un groupe silyle substitué ou non substitué, un atome d'halogène, ou une combinaison de ceux-ci,
R^{b} et R^{c} sont chacun indépendamment présents ou liés l'un à l'autre pour former un noyau,
R^{f} et R^{g} sont chacun indépendamment présents ou liés l'un à l'autre pour former un noyau,
R^{h} et Rⁱ sont chacun indépendamment présents ou liés l'un à l'autre pour former un noyau,
R^{k} et R^{l} sont chacun indépendamment présents ou liés l'un à l'autre pour former un noyau,
R^{m} et Rⁿ sont chacun indépendamment présents ou liés l'un à l'autre pour former un noyau, et
m1 et m2 valent chacun indépendamment 1 à 5.

6. Composé selon l'une quelconque des revendications 1 à 5, la longueur d'onde d'absorption maximale du composé se trouvant dans le spectre de longueur d'onde de 700 nm à 3000 nm.

7. Film comprenant le composé selon l'une quelconque des revendications 1 à 6.

8. Diode photoélectrique comprenant :
une première électrode et une seconde électrode se faisant face à face l'une l'autre, et
une couche organique comprise entre la première électrode et la seconde électrode,
ladite couche organique comprenant un composé représenté par la formule chimique 1 : 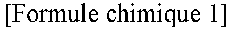 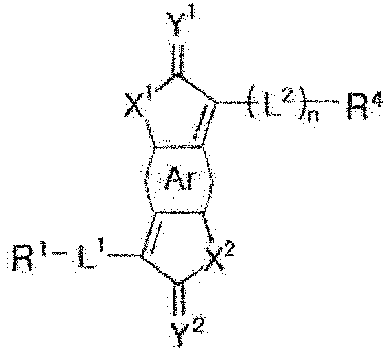
dans laquelle,
X¹ et X² représentent chacun indépendamment un atome O, S, Se ou Te,
Y¹ et Y² représentent chacun indépendamment un atome O, S, Se, Te, ou un groupe C(CN)₂,
Ar représente un noyau aromatique en C6 à C30 substitué ou non substitué, un noyau hétéroaromatique en C3 à C30 substitué ou non substitué, ou une combinaison de ceux-ci,
L¹ et L² représentent chacun indépendamment un groupe hétéroarylène en C3 à C20 substitué ou non substitué,
R¹ représente un groupe -NR²R³, R² et R³ représentant chacun indépendamment un atome d'hydrogène, un groupe alkyle en C1 à C30 substitué ou non substitué, un groupe alcoxy en C1 à C30 substitué ou non substitué, un groupe aryle en C6 à C30 substitué ou non substitué, un groupe hétéroaryle en C3 à C30 substitué ou non substitué, un groupe silyle substitué ou non substitué, un atome d'halogène, ou une combinaison de ceux-ci, R² et R³ étant chacun indépendamment présents ou liés l'un à l'autre pour former un noyau,
R⁴ est représenté par un groupe -NR⁵R⁶,
R⁵ et R⁶ représentant chacun indépendamment un atome d'hydrogène, un groupe alkyle en C1 à C30 substitué ou non substitué, un groupe alcoxy en C1 à C30 substitué ou non substitué, un groupe aryle en C6 à C30 substitué ou non substitué, un groupe hétéroaryle en C3 à C30 substitué ou non substitué, un groupe silyle substitué ou non substitué, un atome d'halogène, ou une combinaison de ceux-ci, R⁵ et R⁶ étant chacun indépendamment présents ou liés l'un à l'autre pour former un noyau, et
n vaut 0 ou 1.

9. Diode photoélectrique selon la revendication 8, Ar représentant un noyau benzène substitué ou non substitué, un noyau naphtalène substitué ou non substitué, un noyau anthracène substitué ou non substitué, un noyau phénanthrène substitué ou non substitué, un noyau tétracène substitué ou non substitué, un noyau pyrène substitué ou non substitué, un noyau thiophène substitué ou non substitué, un noyau furane substitué ou non substitué, un noyau sélénophène substitué ou non substitué, un noyau tellurophène substitué ou non substitué, ou une combinaison d'au moins deux noyaux parmi ceux-ci fusionnés ensemble.

10. Diode photoélectrique selon la revendication 8 ou 9, L¹ et L² comprenant indépendamment au moins l'un des groupes substitués ou non substitués énumérés dans le groupe 1 : 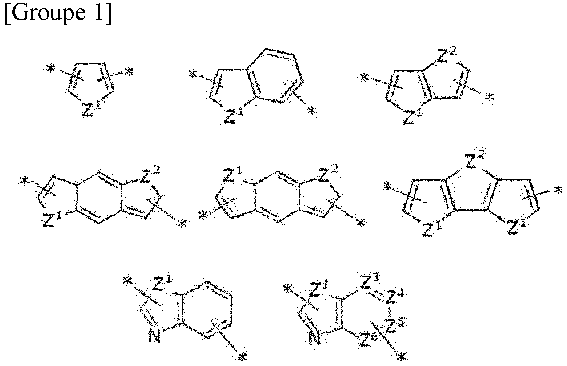 dans lesquels,
Z¹ et Z² représentent chacun indépendamment un atome O, S, Se, Te, un groupe NR^{a} ou SiR^{b}R^{c},
Z³ à Z⁶ représentent chacun indépendamment un atome N ou un groupe CR^{d},
au moins l'un des groupes Z³ à Z⁶ représente un groupe N,
R^{a} à R^{d} représentant chacun indépendamment un atome d'hydrogène, un groupe alkyle en C1 à C30 substitué ou non substitué, un groupe alcényle en C2 à C30 substitué ou non substitué, un groupe alcynyle en C2 à C30 substitué ou non substitué, un groupe aryle en C6 à C30 substitué ou non substitué, un groupe hétéroaryle en C3 à C30 substitué ou non substitué, un groupe amine substitué ou non substitué, un groupe silyle substitué ou non substitué, ou une combinaison de ceux-ci, R^{b} et R^{c} étant chacun indépendamment présents ou liés l'un à l'autre pour former un noyau, et
* représente un point de liaison.

11. Diode photoélectrique selon l'une quelconque des revendications 8 à 10, R¹ étant représenté par l'une des formules chimiques A-1 à A-3 : 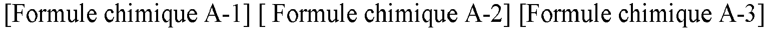 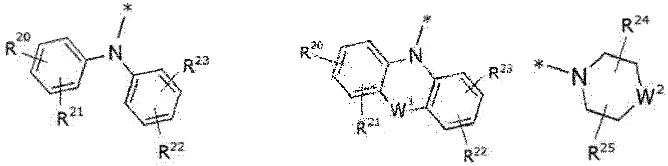
W¹ représentant une liaison simple, un atome O, S, Se, un groupe NR^{e}, CR^{f}R^{g} ou SiR^{h}Rⁱ,
W² représentant un atome O, S, Se, un groupe NR^{e}, CR^{f}R^{g} ou SiR^{h}Rⁱ,
R²⁰ à R²⁵ et R^{e} à Rⁱ représentant chacun indépendamment un atome d'hydrogène, un groupe alkyle en C1 à C30 substitué ou non substitué, un groupe alcényle en C2 à C30 substitué ou non substitué, un groupe alcynyle en C2 à C30 substitué ou non substitué, un groupe aryle en C6 à C30 substitué ou non substitué, un groupe hétéroaryle en C3 à C30 substitué ou non substitué, un groupe amine substitué ou non substitué, un groupe silyle substitué ou non substitué, un atome d'halogène, ou une combinaison de ceux-ci,
R²⁰ et R²¹ étant chacun indépendamment présents ou liés l'un à l'autre pour former un noyau,
R²² et R²³ étant chacun indépendamment présents ou liés l'un à l'autre pour former un noyau,
R²⁴ et R²⁵ étant chacun indépendamment présents ou liés l'un à l'autre pour former un noyau,
R^{f} et R^{g} étant chacun indépendamment présents ou liés l'un à l'autre pour former un noyau,
R^{h} et Rⁱ étant chacun indépendamment présents ou liés l'un à l'autre pour former un noyau,
dans la formule chimique A-1, la formule chimique A-2 ou la formule chimique A-3, au moins un atome d'hydrogène dans la structure de noyau étant éventuellement remplacé par un groupe alkyle en C1 à C30 substitué ou non substitué, un groupe alcényle en C2 à C30 substitué ou non substitué, un groupe alcynyle en C2 à C30 substitué ou non substitué, un groupe aryle en C6 à C30 substitué ou non substitué, un groupe hétéroaryle en C3 à C30 substitué ou non substitué, un groupe silyle substitué ou non substitué, un atome d'halogène, ou une combinaison de ceux-ci,
* représentant un pont de liaison, et
R⁴ étant représenté par l'une des formules chimique B-1 à B-3 : 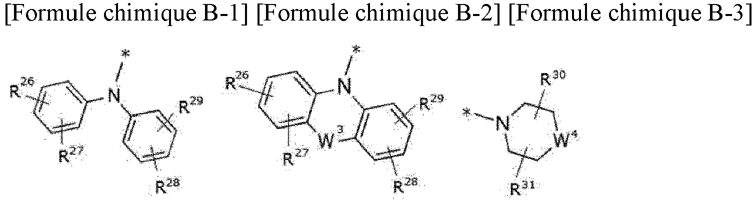
dans lesquelles,
W³ représente une liaison simple, un atome O, S, Se, un groupe NR^{j}, CR^{k}R^{l} ou SiR^{m}Rⁿ,
W⁴ représente un atome O, S, Se, un groupe NR^{j}, CR^{k}R^{l} ou SiR^{m}Rⁿ,
R²⁶ à R³¹ et R^{j} à Rⁿ représentent chacun indépendamment un atome d'hydrogène, un groupe alkyle en C1 à C30 substitué ou non substitué, un groupe alcényle en C2 à C30 substitué ou non substitué, un groupe alcynyle en C2 à C30 substitué ou non substitué, un groupe aryle en C6 à C30 substitué ou non substitué, un groupe hétéroaryle en C3 à C30 substitué ou non substitué, un groupe amine substitué ou non substitué, un groupe silyle substitué ou non substitué, un atome d'halogène, ou une combinaison de ceux-ci,
R²⁶ et R²⁷ sont chacun indépendamment présents ou liés l'un à l'autre pour former un noyau,
R²⁸ et R²⁹ sont chacun indépendamment présents ou liés l'un à l'autre pour former un noyau,
R³⁰ et R³¹ sont chacun indépendamment présents ou liés l'un à l'autre pour former un noyau,
R^{k} et R^{l} sont chacun indépendamment présents ou liés l'un à l'autre pour former un noyau,
R^{m} et Rⁿ sont chacun indépendamment présents ou liés l'un à l'autre pour former un noyau,
dans la formule chimique B-1, la formule chimique B-2 ou la formule chimique B-3, au moins un atome d'hydrogène dans la structure de noyau est éventuellement remplacé par un groupe alkyle en C1 à C30 substitué ou non substitué, un groupe alcényle en C2 à C30 substitué ou non substitué, un groupe alcynyle en C2 à C30 substitué ou non substitué, un groupe aryle en C6 à C30 substitué ou non substitué, un groupe hétéroaryle en C3 à C30 substitué ou non substitué, un groupe silyle substitué ou non substitué, un atome d'halogène, ou une combinaison de ceux-ci, et
* représente un point de liaison.

12. Diode photoélectrique selon l'une quelconque des revendications 8 à 11, ladite couche organique comprenant en outre un fullerène ou un dérivé de fullerène.

13. Diode photoélectrique selon l'une quelconque des revendications 8 à 12, la longueur d'onde d'absorption maximale du composé se trouvant dans le spectre de longueur d'onde de 700 nm à 3000 nm.

14. Capteur organique comprenant la diode photoélectrique selon l'une quelconque des revendications 8 à 13.

15. Dispositif électronique comprenant le capteur organique selon la revendication 14 ou la diode photoélectrique selon l'une quelconque des revendications 8 à 13.
